# FASCICULE DE BREVET EUROPEEN

(11) **EP 2 780 356 B1**
(45) Date de publication et mention de la délivrance du brevet: **07.06.2017**
(21) Numéro de dépôt: 12795560.7
(22) Date de dépôt: 16.11.2012
(51) Int. Cl.: C07K 4/12, C07K 14/47, A61K 38/03, A61K 38/17

(54) **PEPTIDES INHIBITEURS DE L'INTERACTION ENTRE ASF1 ET LES HISTONES, ET LEURS UTILISATIONS**
PEPTIDE ZUR HEMMUNG DER WECHSELWIRKUNG ZWISCHEN ASF1 UND HISTONEN UND IHRE VERWENDUNG
PEPTIDES THAT INHIBIT THE INTERACTION BETWEEN ASF1 AND HISTONES, AND USE THEREOF

(30) Priorité: 18.11.2011 FR 1160536
(43) Date de publication de la demande: 24.09.2014
(73) Titulaire: Commissariat à l'Énergie Atomique et aux Énergies Alternatives, 75015 Paris (FR); Université Pierre et Marie Curie (Paris 6), 75005 Paris (FR)
(72) Inventeur: OCHSENBEIN, Françoise, 91190 Gif Sur Yvette (FR); GUEROIS, Raphaël, 91190 Gif Sur Yvette (FR); GAUBERT, Albane, 92210 Saint Cloud (FR); COURBEYRETTE, Régis, 91300 Massy (FR)
(74) Mandataire: Gallois, Valérie
(86) Numéro de dépôt international: PCT/FR2012/052641
(87) Numéro de publication internationale: WO 2013/072636

(56) Documents cités:
- ANTCZAK ANDREW J ET AL: "Structure of the yeast histone H3-ASF1 interaction: implications for chaperone mechanism, species-specific interactions, and epigenetics", BMC STRUCTURAL BIOLOGY, BIOMED CENTRAL LTD., LONDON, GB, vol. 6, no. 1, 13 décembre 2006 (2006-12-13), page 26, XP021023631, ISSN: 1472-6807, DOI: 10.1186/1472-6807-6-26
- GROTH A ET AL: "Human Asf1 regulates the flow of S phase histones during replicational stress", MOLECULAR CELL, CELL PRESS, CAMBRIDGE, MA, US, vol. 17, no. 2, 21 janvier 2005 (2005-01-21), pages 301-311, XP002603115, ISSN: 1097-2765, DOI: 10.1016/J.MOLCEL.2004.12.018 [extrait le 2005-01-20]
- DE BENEDETTI ARRIGO: "Tousled kinase TLK1B counteracts the effect of Asf1 in inhibition of histone H3â H4 tetramer formation", BMC RESEARCH NOTES, BIOMED CENTRAL LTD, GB, vol. 2, no. 1, 8 juillet 2009 (2009-07-08) , page 128, XP021060715, ISSN: 1756-0500, DOI: 10.1186/1756-0500-2-128

### SEQUENCE LISTING

<110> COMMISSARIAT A L'ENERGIE ATOMIQUE ET AUX ENERGIES ALTERNATIVES Université Pierre et Marie Curie (Paris 6)
<120> Peptides inhibiteurs de l'interaction entre Asf1 et les histones, et leurs utilisations
<130> B1257FR
<160> 82
<170> PatentIn version 3.3
<210> 1
   <211> 13
   <212> PRT
   <213> artificial sequence
<220>
   <223> Formule (I) = E1, mode de réalisation a1
<220>
   <221> MISC_FEATURE
   <222> (1)..(1)
   <223> Xaa est X3, un acide aminé hydrophobe ou R, de préférence A, C, V, P, L, I, M, F, W, R ou Y
<220>
   <221> MISC_FEATURE
   <222> (2)..(2)
   <223> Xaa est X4, étant T, S, P, D, M ou N
<220>
   <221> MISC_FEATURE
   <222> (3)..(3)
   <223> Xaa est X5, un acide aminé quelconque (A, R, D, N, C, G, Q, E, H, I, L, K, M, F, P, S, T, V, W ou Y)
<220>
   <221> MISC_FEATURE
   <222> (5)..(5)
   <223> Xaa est X6, étant Q, E, L, M, A, D ou W
<220>
   <221> MISC_FEATURE
   <222> (6)..(7)
   <223> Xaa sont X7 et X8 , des acides aminés quelconques (A, R, D, N, C, G, Q, E, H, I, L, K, M, F, P, S, T, V, W ou Y)
<220>
   <221> MISC_FEATURE
   <222> (9)..(10)
   <223> Xaa sont X9 et X10, des acides aminés quelconques (A, R, D, N, C, G, Q, E, H, I, L, K, M, F, P, S, T, V, W ou Y)
<220>
   <221> MISC_FEATURE
   <222> (13)..(13)
   <223> Xaa est X11, un acide aminé quelconque (A, R, D, N, C, G, Q, E, H, I, L, K, M, F, P, S, T, V, W ou Y)
<400> 1
   Xaa Xaa Xaa Lys Xaa Xaa Xaa Leu Xaa Xaa Arg Ile Xaa
   1               5                   10
<210> 2
   <211> 15
   <212> PRT
   <213> artificial sequence
<220>
   <223> Formule (I) E1, mode de réalisation a2
<220>
   <221> MISC_FEATURE
   <222> (1)..(1)
   <223> Xaa est X1, étant absent ou un acide aminé quelconque (A, R, D, N, C, G, Q, E, H, I, L, K, M, F, P, S, T, V, W et Y)
<220>
   <221> MISC_FEATURE
   <222> (2)..(2)
   <223> Xaa est X2, étant absent ou étant un acide aminé quelconque (A, R, D, N, C, G, Q, E, H, I, L, K, M, F, P, S, T, V, W et Y)
<220>
   <221> MISC_FEATURE
   <222> (3)..(3)
   <223> Xaa est X3, étant T, S, P, D ou N
<220>
   <221> MISC_FEATURE
   <222> (4)..(5)
   <223> Xaa sont X4 et X5, étant des acides aminés favorables à une structure secondaire en hélice alpha, de préférence s A, R, D, N, C, G, Q, E, H, L, K, M, F, S, W ou Y
<220>
   <221> MISC_FEATURE
   <222> (7)..(7)
   <223> Xaa est X6, étant Q, E, L, M, A, D ou W
<220>
   <221> MISC_FEATURE
   <222> (8)..(9)
   <223> Xaa sont X7 et X8, étant des acides aminés quelconques (A, R, D, N, C, G, Q, E, H, I, L, K, M, F, P, S, T, V, W et Y)
<220>
   <221> MISC_FEATURE
   <222> (11)..(12)
   <223> Xaa sont X9 et X10, étant des acides aminés quelconques (A, R, D, N, C, G, Q, E, H, I, L, K, M, F, P, S, T, V, W et Y)
<220>
   <221> MISC_FEATURE
   <222> (15)..(15)
   <223> Xaa sest X11, étant un acide aminé quelconque (A, R, D, N, C, G, Q, E, H, I, L, K, M, F, P, S, T, V, W et Y)
<400> 2
   Xaa Xaa Xaa Xaa Xaa Lys Xaa Xaa Xaa Leu Xaa Xaa Arg Ile Xaa
   1               5                   10                  15
<210> 3
   <211> 5
   <212> PRT
   <213> artificial sequence
<220>
   <223> Formule (II)= B
<220>
   <221> MISC_FEATURE
   <222> (2)..(2)
   <223> Xaa est X12, étant un acide aminé quelconque (A, R, D, N, C, G, Q, E, H, I, L, K, M, F, P, S, T, V, W et Y)
<220>
   <221> MISC_FEATURE
   <222> (4)..(4)
   <223> Xaa est X13, étant absent ou étant G, A ou S
<220>
   <221> MISC_FEATURE
   <222> (5)..(5)
   <223> Xaa est X14, étant absent ou un acide aminé quelconque (A, R, D, N, C, G, Q, E, H, I, L, K, M, F, P, S, T, V, W et Y)
<400> 3
   Gly Xaa Gly Xaa Xaa
   1               5
<210> 4
   <211> 7
   <212> PRT
   <213> artificial sequence
<220>
   <223> Formule (III) = E2
<220>
   <221> MISC_FEATURE
   <222> (1)..(1)
   <223> Xaa est X15, étant un acide aminé quelconque (A, R, D, N, C, G, Q, E, H, I, L, K, M, F, P, S, T, V, W et Y)
<220>
   <221> MISC_FEATURE
   <222> (2)..(2)
   <223> Xaa est X16, étant T ou V
<220>
   <221> MISC_FEATURE
   <222> (3)..(3)
   <223> Xaa est X17, étant un acide aminé quelconque (A, R, D, N, C, G, Q, E, H, I, L, K, M, F, P, S, T, V, W et Y)
<220>
   <221> MISC_FEATURE
   <222> (4)..(4)
   <223> Xaa est X18, étant un acide aminé quelconque non-chargé positivement (A, D, N, C, G, Q, E, H, I, L, M, F, P, S, T, V, W et Y)
<220>
   <221> MISC_FEATURE
   <222> (6)..(6)
   <223> Xaa est X19, étant un acide aminé hydrophobe, de préférence A, C, V, P, L, I, M, F, W ou Y
<220>
   <221> MISC_FEATURE
   <222> (7)..(7)
   <223> Xaa est X20, étant absent ou un acide aminé quelconque (A, R, D, N, C, G, Q, E, H, I, L, K, M, F, P, S, T, V, W et Y)
<400> 4
   Xaa Xaa Xaa Xaa Gly Xaa Xaa
   1               5
<210> 5
   <211> 25
   <212> PRT
   <213> artificial sequence
<220>
   <223> Formule (IV) = E1 (a1) -B-E2
<220>
   <221> MISC_FEATURE
   <222> (1)..(1)
   <223> Xaa est X3, étant un acide aminé A, C, V, P, L, I, M, F, W, R ou Y, de préférence A, C, V, P, L, I, M, F, W ou Y
<220>
   <221> MISC_FEATURE
   <222> (2)..(2)
   <223> Xaa est X4, étant T, S, P, D, M ou N
<220>
   <221> MISC_FEATURE
   <222> (3)..(3)
   <223> Xaa est X5, étant un acide aminé quelconque (A, R, D, N, C, G, Q, E, H, I, L, K, M, F, P, S, T, V, W et Y)
<220>
   <221> MISC_FEATURE
   <222> (5)..(5)
   <223> Xaa est X6, étant Q, E, L, M, A, D ou W
<220>
   <221> MISC_FEATURE
   <222> (6)..(7)
   <223> Xaa sont X7 et X8, étant des acides aminés quelconques (A, R, D, N, C, G, Q, E, H, I, L, K, M, F, P, S, T, V, W et Y)
<220>
   <221> MISC_FEATURE
   <222> (9)..(10)
   <223> Xaa sont X9 et X10, étant des acides aminés quelconques (A, R, D, N, C, G, Q, E, H, I, L, K, M, F, P, S, T, V, W et Y)
<220>
   <221> MISC_FEATURE
   <222> (13)..(13)
   <223> Xaa est X11, étant un acide aminé quelconque (A, R, D, N, C, G, Q, E, H, I, L, K, M, F, P, S, T, V, W et Y)
<220>
   <221> MISC_FEATURE
   <222> (15)..(15)
   <223> Xaa est X12, étant un acide aminé quelconque (A, R, D, N, C, G, Q, E, H, I, L, K, M, F, P, S, T, V, W et Y)
<220>
   <221> MISC_FEATURE
   <222> (17)..(17)
   <223> Xaa est X13, étant absent ou sélectionné parmi les acides aminés G, A et S
<220>
   <221> MISC_FEATURE
   <222> (18)..(18)
   <223> Xaa est X14, étant absent ou un acide aminé quelconque (A, R, D, N, C, G, Q, E, H, I, L, K, M, F, P, S, T, V, W et Y)
<220>
   <221> MISC_FEATURE
   <222> (19)..(19)
   <223> Xaa est X15, étant un acide aminé quelconque (A, R, D, N, C, G, Q, E, H, I, L, K, M, F, P, S, T, V, W et Y)
<220>
   <221> MISC_FEATURE
   <222> (20)..(20)
   <223> Xaa est X16, étant T ou V
<220>
   <221> MISC_FEATURE
   <222> (21)..(21)
   <223> Xaa est X17, étant un acide aminé quelconque (A, R, D, N, C, G, Q, E, H, I, L, K, M, F, P, S, T, V, W et Y)
<220>
   <221> MISC_FEATURE
   <222> (22)..(22)
   <223> Xaa est X18, étant un acide aminé quelconque non-chargé positivement (A, D, N, C, G, Q, E, H, I, L, M, F, P, S, T, V, W et Y)
<220>
   <221> MISC_FEATURE
   <222> (24)..(24)
   <223> Xaa est X19, étant un acide aminé hydrophobe, de préférence A, C, V, P, L, I, M, F, W ou Y
<220>
   <221> MISC_FEATURE
   <222> (25)..(25)
   <223> Xaa est X20, étant absent ou un acide aminé quelconque (A, R, D, N, C, G, Q, E, H, I, L, K, M, F, P, S, T, V, W et Y)
<400> 5
   Xaa Xaa Xaa Lys Xaa Xaa Xaa Leu Xaa Xaa Arg Ile Xaa Gly Xaa Gly
   1               5                   10                  15
   Xaa Xaa Xaa Xaa Xaa Xaa Gly Xaa Xaa
               20                  25
<210> 6
   <211> 27
   <212> PRT
   <213> artificial sequence
<220>
   <223> Formule (V) = E1 (a2) -B-E2
<220>
   <221> MISC_FEATURE
   <222> (1)..(1)
   <223> Xaa est X1, étant absent ou un acide aminé quelconque (A, R, D, N, C, G, Q, E, H, I, L, K, M, F, P, S, T, V, W et Y)
<220>
   <221> MISC_FEATURE
   <222> (2)..(2)
   <223> Xaa est X2, étant absent ou un acide aminé quelconque (A, R, D, N, C, G, Q, E, H, I, L, K, M, F, P, S, T, V, W et Y)
<220>
   <221> MISC_FEATURE
   <222> (3)..(3)
   <223> Xaa est X3, étant T, S, P, D ou N
<220>
   <221> MISC_FEATURE
   <222> (4)..(5)
   <223> Xaa sont X4 et X5, étant des acides aminés favorables à une structure secondaire en hélice alpha, de préférence A, R, D, N, C, G, Q, E, H, L, K, M, F, S, W ou Y
<220>
   <221> MISC_FEATURE
   <222> (7)..(7)
   <223> Xaa est X6, étant Q, E, L, M, A, D ou W
<220>
   <221> MISC_FEATURE
   <222> (8)..(9)
   <223> Xaa sont X7 et X8, étant des acides aminés quelconques (A, R, D, N, C, G, Q, E, H, I, L, K, M, F, P, S, T, V, W et Y)
<220>
   <221> MISC_FEATURE
   <222> (11)..(12)
   <223> Xaa sont X9 et X10, étant des acides aminés quelconques (A, R, D, N, C, G, Q, E, H, I, L, K, M, F, P, S, T, V, W et Y)
<220>
   <221> MISC_FEATURE
   <222> (15)..(15)
   <223> Xaa est X11, étant un acide aminé quelconque (A, R, D, N, C, G, Q, E, H, I, L, K, M, F, P, S, T, V, W et Y)
<220>
   <221> MISC_FEATURE
   <222> (17)..(17)
   <223> Xaa est X12, étant un acide aminé quelconque (A, R, D, N, C, G, Q, E, H, I, L, K, M, F, P, S, T, V, W et Y)
<220>
   <221> MISC_FEATURE
   <222> (19)..(19)
   <223> Xaa est X13, étant absent ou un acide aminé G, A ou S
<220>
   <221> MISC_FEATURE
   <222> (20)..(20)
   <223> Xaa est X14, étant absent ou un acide aminé quelconque (A, R, D, N, C, G, Q, E, H, I, L, K, M, F, P, S, T, V, W et Y)
<220>
   <221> MISC_FEATURE
   <222> (21)..(21)
   <223> Xaa est X15, étant un acide aminé quelconque (A, R, D, N, C, G, Q, E, H, I, L, K, M, F, P, S, T, V, W et Y)
<220>
   <221> MISC_FEATURE
   <222> (22)..(22)
   <223> Xaa est X16, étant T ou V
<220>
   <221> MISC_FEATURE
   <222> (23)..(23)
   <223> Xaa est X17, étant un acide aminé quelconque (A, R, D, N, C, G, Q, E, H, I, L, K, M, F, P, S, T, V, W et Y)
<220>
   <221> MISC_FEATURE
   <222> (24)..(24)
   <223> Xaa est X18, étant un acide aminé quelconque non-chargé positivement (A, D, N, C, G, Q, E, H, I, L, M, F, P, S, T, V, W ou Y)
<220>
   <221> MISC_FEATURE
   <222> (26)..(26)
   <223> Xaa est X19, étant un acide aminé hydrophobe, de préférence A, C, V, P, L, I, M, F, W ou Y
<220>
   <221> MISC_FEATURE
   <222> (27)..(27)
   <223> Xaa est X20, étant absent ou un acide aminé quelconque (A, R, D, N, C, G, Q, E, H, I, L, K, M, F, P, S, T, V, W et Y)
<400> 6
   Xaa Xaa Xaa Xaa Xaa Lys Xaa Xaa Xaa Leu Xaa Xaa Arg Ile Xaa Gly
   1               5                   10                  15
   Xaa Gly Xaa Xaa Xaa Xaa Xaa Xaa Gly Xaa Xaa
               20                  25
<210> 7
   <211> 13
   <212> PRT
   <213> artificial sequence
<220>
   <223> Formule (I) = E1, mode de réalisation préféré de a1
<220>
   <221> MISC_FEATURE
   <222> (1)..(1)
   <223> Xaa est X3, étant un acide aminé hydrophobe s(A, C, V, P, L, I, M, F, W ou Y)
<220>
   <221> MISC_FEATURE
   <222> (2)..(2)
   <223> Xaa est X4, étant T, S, P, D ou N
<220>
   <221> MISC_FEATURE
   <222> (3)..(3)
   <223> Xaa est X5, étant un acide aminé quelconque, (A, R, D, N, C, G, Q, E, H, I, L, K, M, F, P, S, T, V, W ou Y)
<220>
   <221> MISC_FEATURE
   <222> (5)..(5)
   <223> Xaa est X6, étant Q, E, L, M, A, ou W
<220>
   <221> MISC_FEATURE
   <222> (6)..(7)
   <223> Xaa sont X7 et X8, étant des acides aminés favorables à une structure secondaire en hélice alpha, de préférence A, R, D, N, C, G, Q, E, H, L, K, M, F, S, W ou Y
<220>
   <221> MISC_FEATURE
   <222> (9)..(9)
   <223> Xaa est X9, étant unacide aminé favorable à une structure secondaire en hélice alpha, de préférence A, R, D, N, C, G, Q, E, H, L, K, M, F, S, W ou Y
<220>
   <221> MISC_FEATURE
   <222> (10)..(10)
   <223> Xaa est X10, étant un acide aminé quelconque, (A, R, D, N, C, G, Q, E, H, I, L, K, M, F, P, S, T, V, W ou Y)
<220>
   <221> MISC_FEATURE
   <222> (13)..(13)
   <223> Xaa est X11, étant un acide aminé quelconque, (A, R, D, N, C, G, Q, E, H, I, L, K, M, F, P, S, T, V, W ou Y)
<400> 7
   Xaa Xaa Xaa Lys Xaa Xaa Xaa Leu Xaa Xaa Arg Ile Xaa
   1               5                   10
<210> 8
   <211> 15
   <212> PRT
   <213> artificial sequence
<220>
   <223> Formule (I) = E1, mode de réalisation préféré de a2
<220>
   <221> MISC_FEATURE
   <222> (1)..(2)
   <223> Xaa sont X1 et X2, étant des acides aminés quelconques, (A, R, D, N, C, G, Q, E, H, I, L, K, M, F, P, S, T, V, W ou Y)
<220>
   <221> MISC_FEATURE
   <222> (3)..(3)
   <223> Xaa est X3, étant T, S, P, D ou N
<220>
   <221> MISC_FEATURE
   <222> (4)..(5)
   <223> Xaa sont X4 et X5, étant des acides aminés favorables à une structure secondaire en hélice alpha, de préférence A, R, D, N, C, G, Q, E, H, L, K, M, F, S, W ou Y
<220>
   <221> MISC_FEATURE
   <222> (7)..(7)
   <223> Xaa est X6, étant Q, E, L, M, A, ou W
<220>
   <221> MISC_FEATURE
   <222> (8)..(9)
   <223> Xaa sont X7 et X8, étant des acides aminés favorables à une structure secondaire en hélice alpha, de préférence A, R, D, N, C, G, Q, E, H, L, K, M, F, S, W ou Y
<220>
   <221> MISC_FEATURE
   <222> (11)..(11)
   <223> Xaa est X9, étant un acide aminé favorable à une structure secondaire en hélice alpha, de préférence A, R, D, N, C, G, Q, E, H, L, K, M, F, S, W ou Y
<220>
   <221> MISC_FEATURE
   <222> (12)..(12)
   <223> Xaa est X10, étant un acide aminé quelconque, (A, R, D, N, C, G, Q, E, H, I, L, K, M, F, P, S, T, V, W ou Y)
<220>
   <221> MISC_FEATURE
   <222> (15)..(15)
   <223> Xaa est X11, étant un acide aminé quelconque, (A, R, D, N, C, G, Q, E, H, I, L, K, M, F, P, S, T, V, W ou Y)
<400> 8
   Xaa Xaa Xaa Xaa Xaa Lys Xaa Xaa Xaa Leu Xaa Xaa Arg Ile Xaa
   1               5                   10                  15
<210> 9
   <211> 25
   <212> PRT
   <213> artificial sequence
<220>
   <223> Formule (IV) préférée = E1 (a1) -B-E2
<220>
   <221> MISC_FEATURE
   <222> (1)..(1)
   <223> Xaa est X3, étant un acide aminé hydrophobe sélectionné parmi le groupe consistant en A, C, V, P, L, I, M, F, W et Y
<220>
   <221> MISC_FEATURE
   <222> (2)..(2)
   <223> Xaa est X4, étant T, S, P, D ou N
<220>
   <221> MISC_FEATURE
   <222> (3)..(3)
   <223> Xaa est X5, étant un acide aminé quelconque (A, R, D, N, C, G, Q, E, H, I, L, K, M, F, P, S, T, V, W et Y)
<220>
   <221> MISC_FEATURE
   <222> (5)..(5)
   <223> Xaa est X6, étant Q, E, L, M, A, ou W
<220>
   <221> MISC_FEATURE
   <222> (6)..(7)
   <223> Xaa sont X7 et X8, étant des acides aminés favorables à une structure secondaire en hélice alpha, de préférence A, R, D, N, C, G, Q, E, H, L, K, M, F, S, W ou Y
<220>
   <221> MISC_FEATURE
   <222> (9)..(9)
   <223> Xaa est X9, étant un acide aminé favorable à une structure secondaire en hélice alpha, de préférence A, R, D, N, C, G, Q, E, H, L, K, M, F, S, W ou Y
<220>
   <221> MISC_FEATURE
   <222> (10)..(10)
   <223> Xaa est X10, étant un acide aminé quelconque, (A, R, D, N, C, G, Q, E, H, I, L, K, M, F, P, S, T, V, W ou Y)
<220>
   <221> MISC_FEATURE
   <222> (13)..(13)
   <223> Xaa est X11, étant un acide aminé quelconque, (A, R, D, N, C, G, Q, E, H, I, L, K, M, F, P, S, T, V, W ou Y)
<220>
   <221> MISC_FEATURE
   <222> (15)..(15)
   <223> Xaa est X12, étant un acide aminé quelconque (A, R, D, N, C, G, Q, E, H, I, L, K, M, F, P, S, T, V, W et Y)
<220>
   <221> MISC_FEATURE
   <222> (17)..(17)
   <223> Xaa est X13, étant absent ou sélectionné parmi les acides aminés G, A et S
<220>
   <221> MISC_FEATURE
   <222> (18)..(18)
   <223> Xaa est X14, étant absent ou un acide aminé quelconque (A, R, D, N, C, G, Q, E, H, I, L, K, M, F, P, S, T, V, W et Y)
<220>
   <221> MISC_FEATURE
   <222> (19)..(19)
   <223> Xaa est X15, étant A, T, V, I ou R
<220>
   <221> MISC_FEATURE
   <222> (20)..(20)
   <223> Xaa est X16, étant T ou V
<220>
   <221> MISC_FEATURE
   <222> (21)..(21)
   <223> Xaa est X17, étant un acide aminé quelconque, (A, R, D, N, C, G, Q, E, H, I, L, K, M, F, P, S, T, V, W ou Y)
<220>
   <221> MISC_FEATURE
   <222> (22)..(22)
   <223> Xaa est X18, étant un acide aminé quelconque non-chargé positivement (A, D, N, C, G, Q, E, H, I, L, M, F, P, S, T, V, W et Y)
<220>
   <221> MISC_FEATURE
   <222> (24)..(24)
   <223> Xaa est X19, étant uun acide aminé hydrophobe, de préférence A, C, V, P, L, I, M, F, W ou Y
<220>
   <221> MISC_FEATURE
   <222> (25)..(25)
   <223> Xaa est X20, étant absent ou étant un acide aminé quelconque, (A, R, D, N, C, G, Q, E, H, I, L, K, M, F, P, S, T, V, W ou Y)
<400> 9
   Xaa Xaa Xaa Lys Xaa Xaa Xaa Leu Xaa Xaa Arg Ile Xaa Gly Xaa Gly
   1               5                   10                  15
   Xaa Xaa Xaa Xaa Xaa Xaa Gly Xaa Xaa
               20                  25
<210> 10
   <211> 27
   <212> PRT
   <213> artificial sequence
<220>
   <223> Formule (V) préférée = E1 (a2) -B-E2
<220>
   <221> MISC_FEATURE
   <222> (1)..(2)
   <223> Xaa sont X1 et X2, étant des acides aminés quelconques, (A, R, D, N, C, G, Q, E, H, I, L, K, M, F, P, S, T, V, W ou Y)
<220>
   <221> MISC_FEATURE
   <222> (3)..(3)
   <223> Xaa est X3, étant T, S, P, D ou N
<220>
   <221> MISC_FEATURE
   <222> (4)..(5)
   <223> Xaa sont X4 et X5, étant des acides aminés favorables à une structure secondaire en hélice alpha, de préférence A, R, D, N, C, G, Q, E, H, L, K, M, F, S, W ou Y
<220>
   <221> MISC_FEATURE
   <222> (7)..(7)
   <223> Xaa est X6, étant Q, E, L, M, A, ou W
<220>
   <221> MISC_FEATURE
   <222> (8)..(9)
   <223> Xaa sont X7 et X8, étant des acides aminés favorables à une structure secondaire en hélice alpha, de préférence A, R, D, N, C, G, Q, E, H, L, K, M, F, S, W ou Y
<220>
   <221> MISC_FEATURE
   <222> (11)..(11)
   <223> Xaa est X9, étant un acide aminé favorable à une structure secondaire en hélice alpha, de préférence A, R, D, N, C, G, Q, E, H, L, K, M, F, S, W ou Y
<220>
   <221> MISC_FEATURE
   <222> (12)..(12)
   <223> Xaa est X10, étant un acide aminé quelconque, (A, R, D, N, C, G, Q, E, H, I, L, K, M, F, P, S, T, V, W ou Y)
<220>
   <221> MISC_FEATURE
   <222> (15)..(15)
   <223> Xaa est X11, étant un acide aminé quelconque, (A, R, D, N, C, G, Q, E, H, I, L, K, M, F, P, S, T, V, W ou Y)
<220>
   <221> MISC_FEATURE
   <222> (17)..(17)
   <223> Xaa est X12, étant un acide aminé quelconque (A, R, D, N, C, G, Q, E, H, I, L, K, M, F, P, S, T, V, W et Y)
<220>
   <221> MISC_FEATURE
   <222> (19)..(19)
   <223> Xaa est X13, étant absent ou sélectionné parmi les acides aminés G, A et S
<220>
   <221> MISC_FEATURE
   <222> (20)..(20)
   <223> Xaa est X14, étant absent ou un acide aminé quelconque (A, R, D, N, C, G, Q, E, H, I, L, K, M, F, P, S, T, V, W et Y)
<220>
   <221> MISC_FEATURE
   <222> (21)..(21)
   <223> Xaa est X15, étant A, T, V, I ou R
<220>
   <221> MISC_FEATURE
   <222> (22)..(22)
   <223> Xaa est X16, étant T ou V
<220>
   <221> MISC_FEATURE
   <222> (23)..(23)
   <223> Xaa est X17, étant un acide aminé quelconque (A, R, D, N, C, G, Q, E, H, I, L, K, M, F, P, S, T, V, W et Y)
<220>
   <221> MISC_FEATURE
   <222> (24)..(24)
   <223> Xaa est X18, étant un acide aminé quelconque non-chargé positivement (A, D, N, C, G, Q, E, H, I, L, M, F, P, S, T, V, W et Y)
<220>
   <221> MISC_FEATURE
   <222> (26)..(26)
   <223> Xaa est X19, étant un acide aminé hydrophobe, de préférence s A, C, V, P, L, I, M, F, W ou Y
<220>
   <221> MISC_FEATURE
   <222> (27)..(27)
   <223> Xaa est X20, étant un acide aminé quelconque (A, R, D, N, C, G, Q, E, H, I, L, K, M, F, P, S, T, V, W et Y)
<220>
   <221> MISC_FEATURE
   <222> (27)..(27)
   <223> Xaa est X20, étant absent ou un acide aminé quelconque (A, R, D, N, C, G, Q, E, H, I, L, K, M, F, P, S, T, V, W et Y)
<400> 10
   Xaa Xaa Xaa Xaa Xaa Lys Xaa Xaa Xaa Leu Xaa Xaa Arg Ile Xaa Gly
   1               5                   10                  15
   Xaa Gly Xaa Xaa Xaa Xaa Xaa Xaa Gly Xaa Xaa
               20                  25
<210> 11
   <211> 25
   <212> PRT
   <213> artificial sequence
<220>
   <223> Formule (IV) préférée = E1 (a1) -B-E2
<220>
   <221> MISC_FEATURE
   <222> (1)..(1)
   <223> Xaa est X3, étant I, R ou L
<220>
   <221> MISC_FEATURE
   <222> (2)..(2)
   <223> Xaa est X4, étant T ou M
<220>
   <221> MISC_FEATURE
   <222> (5)..(5)
   <223> Xaa est X6, étant E ou D
<220>
   <221> MISC_FEATURE
   <222> (6)..(6)
   <223> Xaa est X7, étant R, A, I ou E
<220>
   <221> MISC_FEATURE
   <222> (7)..(7)
   <223> Xaa est X8, étant R, Q ou E
<220>
   <221> MISC_FEATURE
   <222> (9)..(9)
   <223> Xaa est X9, étant R ou A
<220>
   <221> MISC_FEATURE
   <222> (13)..(13)
   <223> Xaa est X11, étant R ou E
<220>
   <221> MISC_FEATURE
   <222> (15)..(15)
   <223> Xaa est X12, étant A ou P
<220>
   <221> MISC_FEATURE
   <222> (17)..(17)
   <223> Xaa est X13, étant absent ou sélectionné parmi les acides aminés G, A et S
<220>
   <221> MISC_FEATURE
   <222> (18)..(18)
   <223> Xaa est X14, étant absent ou S
<220>
   <221> MISC_FEATURE
   <222> (19)..(19)
   <223> Xaa est X15, étant R ou V
<220>
   <221> MISC_FEATURE
   <222> (20)..(20)
   <223> Xaa est X16, étant T ou V
<220>
   <221> MISC_FEATURE
   <222> (22)..(22)
   <223> Xaa est X18, étant Y, N ou D
<220>
   <221> MISC_FEATURE
   <222> (24)..(24)
   <223> Xaa est X19, étant F, M, A ou Q
<220>
   <221> MISC_FEATURE
   <222> (25)..(25)
   <223> Xaa est X20, étant G, Q ou N
<400> 11
   Xaa Xaa Pro Lys Xaa Xaa Xaa Leu Xaa Arg Arg Ile Xaa Gly Xaa Gly
   1               5                   10                  15
   Xaa Xaa Xaa Xaa Leu Xaa Gly Xaa Xaa
               20                  25
<210> 12
   <211> 27
   <212> PRT
   <213> artificial sequence
<220>
   <223> Formule (V) préférée = E1 (a2) -B-E2
<220>
   <221> MISC_FEATURE
   <222> (4)..(4)
   <223> Xaa est X4, étant E ou R
<220>
   <221> MISC_FEATURE
   <222> (17)..(17)
   <223> Xaa est X12, étant A ou P
<220>
   <221> MISC_FEATURE
   <222> (19)..(19)
   <223> Xaa est X13, étant absent ou sélectionné parmi les acides aminés G, A et S
<220>
   <221> MISC_FEATURE
   <222> (20)..(20)
   <223> Xaa est X14, étant absent ou S
<220>
   <221> MISC_FEATURE
   <222> (21)..(21)
   <223> Xaa est X15, étant R ou V
<220>
   <221> MISC_FEATURE
   <222> (22)..(22)
   <223> Xaa est X16, étant T ou V
<220>
   <221> MISC_FEATURE
   <222> (24)..(24)
   <223> Xaa est X18, étant Y, N ou D
<220>
   <221> MISC_FEATURE
   <222> (26)..(26)
   <223> Xaa est X19, étant F, M, A ou Q
<220>
   <221> MISC_FEATURE
   <222> (27)..(27)
   <223> Xaa est X20, étant G, Q ou N
<400> 12
   Ala Ser Thr Xaa Arg Lys Trp Ala Glu Leu Ala Arg Arg Ile Arg Gly
   1               5                   10                  15
   Xaa Gly Xaa Xaa Xaa Xaa Leu Xaa Gly Xaa Xaa
               20                  25
<210> 13
   <211> 24
   <212> PRT
   <213> artificial sequence
<220>
   <223> élément E1-B-E2
<400> 13
   Ile Met Pro Lys Asp Ile Gln Leu Ala Arg Arg Ile Arg Gly Ala Gly
   1               5                   10                  15
   Gly Arg Thr Leu Tyr Gly Phe Gly
               20
<210> 14
   <211> 25
   <212> PRT
   <213> artificial sequence
<220>
   <223> élément E1-B-E2
<400> 14
   Ile Met Pro Lys Asp Ile Gln Leu Ala Arg Arg Ile Arg Gly Ala Gly
   1               5                   10                  15
   Ala Ser Arg Thr Leu Tyr Gly Phe Gly
               20                  25
<210> 15
   <211> 24
   <212> PRT
   <213> artificial sequence
<220>
   <223> élément E1-B-E2
<400> 15
   Ile Thr Pro Lys Glu Ala Gln Leu Ala Arg Arg Ile Arg Gly Ala Gly
   1               5                   10                  15
   Gly Arg Thr Leu Asn Gly Phe Gly
               20
<210> 16
   <211> 25
   <212> PRT
   <213> artificial sequence
<220>
   <223> élément E1-B-E2
<400> 16
   Ile Thr Pro Lys Glu Ala Gln Leu Ala Arg Arg Ile Arg Gly Ala Gly
   1               5                   10                  15
   Ala Ser Arg Thr Leu Asn Gly Phe Gly
               20                  25
<210> 17
   <211> 24
   <212> PRT
   <213> artificial sequence
<220>
   <223> élément E1-B-E2
<400> 17
   Leu Thr Pro Lys Glu Ala Glu Leu Ala Arg Arg Ile Arg Gly Ala Gly
   1               5                   10                  15
   Gly Arg Thr Leu Asn Gly Phe Gly
               20
<210> 18
   <211> 25
   <212> PRT
   <213> artificial sequence
<220>
   <223> élément E1-B-E2
<400> 18
   Leu Thr Pro Lys Glu Ala Glu Leu Ala Arg Arg Ile Arg Gly Ala Gly
   1               5                   10                  15
   Ala Ser Arg Thr Leu Asn Gly Phe Gly
               20                  25
<210> 19
   <211> 25
   <212> PRT
   <213> artificial sequence
<220>
   <223> élément E1-B-E2
<400> 19
   Ile Thr Pro Lys Glu Ala Gln Leu Ala Arg Arg Ile Glu Gly Ala Gly
   1               5                   10                  15
   Ala Ser Val Thr Leu Asn Gly Phe Gly
               20                  25
<210> 20
   <211> 25
   <212> PRT
   <213> artificial sequence
<220>
   <223> élément E1-B-E2
<400> 20
   Leu Thr Pro Lys Glu Ala Glu Leu Ala Arg Arg Ile Glu Gly Ala Gly
   1               5                   10                  15
   Ala Ser Val Thr Leu Asn Gly Phe Gly
               20                  25
<210> 21
   <211> 25
   <212> PRT
   <213> artificial sequence
<220>
   <223> élément E1-B-E2
<400> 21
   Ile Thr Pro Lys Glu Glu Gln Leu Arg Arg Arg Ile Glu Gly Ala Gly
   1               5                   10                  15
   Ala Ser Val Thr Leu Asn Gly Phe Gly
               20                  25
<210> 22
   <211> 24
   <212> PRT
   <213> artificial sequence
<220>
   <223> élément E1-B-E2
<400> 22
   Ile Thr Pro Lys Glu Ala Gln Leu Ala Arg Arg Ile Arg Gly Ala Gly
   1               5                   10                  15
   Gly Val Thr Leu Asn Gly Phe Gly
               20
<210> 23
   <211> 24
   <212> PRT
   <213> artificial sequence
<220>
   <223> élément E1-B-E2
<400> 23
   Leu Thr Pro Lys Glu Ala Glu Leu Ala Arg Arg Ile Arg Gly Ala Gly
   1               5                   10                  15
   Gly Val Thr Leu Asn Gly Phe Gly
               20
<210> 24
   <211> 23
   <212> PRT
   <213> artificial sequence
<220>
   <223> élément E1-B-E2
<400> 24
   Leu Thr Pro Lys Glu Ala Glu Leu Ala Arg Arg Ile Arg Gly Ala Gly
   1               5                   10                  15
   Arg Thr Leu Asn Gly Phe Gly
               20
<210> 25
   <211> 24
   <212> PRT
   <213> artificial sequence
<220>
   <223> élément E1-B-E2
<400> 25
   Leu Thr Pro Lys Glu Ala Glu Leu Ala Arg Arg Ile Arg Gly Ala Gly
   1               5                   10                  15
   Gly Arg Thr Leu Asn Gly Phe Gly
               20
<210> 26
   <211> 25
   <212> PRT
   <213> artificial sequence
<220>
   <223> élément E1-B-E2
<400> 26
   Leu Thr Pro Lys Glu Ala Glu Leu Ala Arg Arg Ile Arg Gly Ala Gly
   1               5                   10                  15
   Ala Ser Arg Thr Leu Asn Gly Phe Gly
               20                  25
<210> 27
   <211> 24
   <212> PRT
   <213> artificial sequence
<220>
   <223> élément E1-B-E2
<400> 27
   Arg Thr Pro Lys Glu Arg Arg Leu Ala Arg Arg Ile Arg Gly Ala Gly
   1               5                   10                  15
   Gly Arg Thr Leu Asn Gly Phe Gly
               20
<210> 28
   <211> 24
   <212> PRT
   <213> artificial
<220>
   <223> élément E1-B-E2
<400> 28
   Arg Thr Pro Lys Glu Ala Arg Leu Ala Arg Arg Ile Arg Gly Ala Gly
   1               5                   10                  15
   Gly Arg Thr Leu Asn Gly Phe Gly
               20
<210> 29
   <211> 24
   <212> PRT
   <213> artificial sequence
<220>
   <223> élément E1-B-E2
<400> 29
   Leu Thr Pro Lys Glu Ala Glu Leu Ala Arg Arg Ile Arg Gly Ala Gly
   1               5                   10                  15
   Gly Val Thr Tyr Asp Gly Phe Gly
               20
<210> 30
   <211> 24
   <212> PRT
   <213> artificial sequence
<220>
   <223> élément E1-B-E2
<400> 30
   Leu Thr Pro Lys Glu Ala Glu Leu Ala Arg Arg Ile Arg Gly Ala Gly
   1               5                   10                  15
   Gly Val Thr Leu Asn Gly Ala Asn
               20
<210> 31
   <211> 25
   <212> PRT
   <213> artificial sequence
<220>
   <223> élément E1-B-E2
<400> 31
   Ser Thr Glu Arg Lys Trp Ala Glu Leu Ala Arg Arg Ile Arg Gly Ala
   1               5                   10                  15
   Gly Gly Val Thr Leu Asn Gly Phe Gly
               20                  25
<210> 32
   <211> 26
   <212> PRT
   <213> artificial sequence
<220>
   <223> élément E1-B-E2
<400> 32
   Ala Ser Thr Glu Arg Lys Trp Ala Glu Leu Ala Arg Arg Ile Arg Gly
   1               5                   10                  15
   Ala Gly Gly Val Thr Leu Asn Gly Phe Gly
               20                  25
<210> 33
   <211> 26
   <212> PRT
   <213> artificial sequence
<220>
   <223> élément E1-B-E2
<400> 33
   Ala Ser Thr Arg Arg Lys Trp Ala Glu Leu Ala Arg Arg Ile Arg Gly
   1               5                   10                  15
   Ala Gly Gly Val Thr Leu Asn Gly Phe Gly
               20                  25
<210> 34
   <211> 9
   <212> PRT
   <213> Artificial sequence
<220>
   <223> peptide Tat
<400> 34
   Arg Lys Lys Arg Arg Gln Arg Arg Arg
   1               5
<210> 35
   <211> 16
   <212> PRT
   <213> artificial sequence
<220>
   <223> peptide d'antennapedia
<400> 35
   Arg Gln Ile Lys Ile Trp Phe Gln Asn Arg Arg Met Lys Trp Lys Lys
   1               5                   10                  15
<210> 36
   <211> 9
   <212> PRT
   <213> artificial sequence
<220>
   <223> peptide polyarginine
<400> 36
   Arg Arg Arg Arg Arg Arg Arg Arg Arg
   1               5
<210> 37
   <211> 15
   <212> PRT
   <213> artificial sequence
<220>
   <223> peptide riche en arginine
<400> 37
   Arg Arg Pro Arg Arg Pro Arg Arg Pro Arg Arg Pro Arg Arg Pro
   1               5                   10                  15
<210> 38
   <211> 29
   <212> PRT
   <213> artificial sequence
<220>
   <223> peptide inhibiteur du entre Asf1 et H3-H4 - peptide i4_1
<400> 38
   Gly Ala Met Gly Thr Ile Met Pro Lys Asp Ile Gln Leu Ala Arg Arg
   1               5                   10                  15
   Ile Arg Gly Ala Gly Gly Arg Thr Leu Tyr Gly Phe Gly
               20                  25
<210> 39
   <211> 30
   <212> PRT
   <213> artificial sequence
<220>
   <223> peptide inhibiteur du entre Asf1 et H3-H4
<400> 39
   Gly Ala Met Gly Thr Ile Met Pro Lys Asp Ile Gln Leu Ala Arg Arg
   1               5                   10                  15
   Ile Arg Gly Ala Gly Ala Ser Arg Thr Leu Tyr Gly Phe Gly
               20                  25                  30
<210> 40
   <211> 29
   <212> PRT
   <213> artificial sequence
<220>
   <223> peptide inhibiteur du entre Asf1 et H3-H4- peptide i4_2
<400> 40
   Gly Ala Met Gly Thr Ile Thr Pro Lys Glu Ala Gln Leu Ala Arg Arg
   1               5                   10                  15
   Ile Arg Gly Ala Gly Gly Arg Thr Leu Asn Gly Phe Gly
               20                  25
<210> 41
   <211> 30
   <212> PRT
   <213> artificial sequence
<220>
   <223> peptide inhibiteur du entre Asf1 et H3-H4
<400> 41
   Gly Ala Met Gly Thr Ile Thr Pro Lys Glu Ala Gln Leu Ala Arg Arg
   1               5                   10                  15
   Ile Arg Gly Ala Gly Ala Ser Arg Thr Leu Asn Gly Phe Gly
               20                  25                  30
<210> 42
   <211> 29
   <212> PRT
   <213> artificial sequence
<220>
   <223> peptide inhibiteur du entre Asf1 et H3-H4- peptide i4
<400> 42
   Gly Ala Met Gly Thr Leu Thr Pro Lys Glu Ala Glu Leu Ala Arg Arg
   1               5                   10                  15
   Ile Arg Gly Ala Gly Gly Arg Thr Leu Asn Gly Phe Gly
               20                  25
<210> 43
   <211> 30
   <212> PRT
   <213> artificial
<220>
   <223> peptide inhibiteur du entre Asf1 et H3-H4
<400> 43
   Gly Ala Met Gly Thr Leu Thr Pro Lys Glu Ala Glu Leu Ala Arg Arg
   1 5 10 15
   Ile Arg Gly Ala Gly Ala Ser Arg Thr Leu Asn Gly Phe Gly
   20 25 30
<210> 44
   <211> 30
   <212> PRT
   <213> artificial sequence
<220>
   <223> peptide inhibiteur du entre Asf1 et H3-H4
<400> 44
   Gly Ala Met Gly Thr Ile Thr Pro Lys Glu Ala Gln Leu Ala Arg Arg
   1 5 10 15
   Ile Glu Gly Ala Gly Ala Ser Val Thr Leu Asn Gly Phe Gly
   20 25 30
<210> 45
   <211> 30
   <212> PRT
   <213> artificial sequence
<220>
   <223> peptide inhibiteur du entre Asf1 et H3-H4
<400> 45
   Gly Ala Met Gly Thr Leu Thr Pro Lys Glu Ala Glu Leu Ala Arg Arg
   1 5 10 15
   Ile Glu Gly Ala Gly Ala Ser Val Thr Leu Asn Gly Phe Gly
   20 25 30
<210> 46
   <211> 30
   <212> PRT
   <213> artificial sequence
<220>
   <223> peptide inhibiteur du entre Asf1 et H3-H4
<400> 46
   Gly Ala Met Gly Thr Ile Thr Pro Lys Glu Glu Gln Leu Arg Arg Arg
   1 5 10 15
   Ile Glu Gly Ala Gly Ala Ser Val Thr Leu Asn Gly Phe Gly
   20 25 30
<210> 47
   <211> 29
   <212> PRT
   <213> artificial sequence
<220>
   <223> peptide inhibiteur du entre Asf1 et H3-H4-peptide i42V
<400> 47
   Gly Ala Met Gly Thr Ile Thr Pro Lys Glu Ala Gln Leu Ala Arg Arg
   1               5                   10                  15
   Ile Arg Gly Ala Gly Gly Val Thr Leu Asn Gly Phe Gly
               20                  25
<210> 48
   <211> 29
   <212> PRT
   <213> artificial sequence
<220>
   <223> peptide inhibiteur du entre Asf1 et H3-H4-peptide i4V
<400> 48
   Gly Ala Met Gly Thr Leu Thr Pro Lys Glu Ala Glu Leu Ala Arg Arg
   1               5                   10                  15
   Ile Arg Gly Ala Gly Gly Val Thr Leu Asn Gly Phe Gly
               20                  25
<210> 49
   <211> 28
   <212> PRT
   <213> artificial sequence
<220>
   <223> peptide inhibiteur du entre Asf1 et H3-H4
<400> 49
   Gly Ala Met Gly Thr Leu Thr Pro Lys Glu Ala Glu Leu Ala Arg Arg
   1               5                   10                  15
   Ile Arg Gly Ala Gly Arg Thr Leu Asn Gly Phe Gly
               20                  25
<210> 50
   <211> 46
   <212> PRT
   <213> artificial
<220>
   <223> peptide inhibiteur du entre Asf1 et H3-H4-peptide RRP5-i42V-CA
<400> 50
   Arg Arg Pro Arg Arg Pro Arg Arg Pro Arg Arg Pro Arg Arg Pro Gly
   1               5                   10                  15
   Ala Met Gly Thr Ile Thr Pro Lys Glu Ala Gln Leu Ala Arg Arg Ile
               20                  25                  30
   Arg Gly Ala Gly Gly Val Thr Leu Asn Gly Phe Gly Cys Ala
           35                  40                  45
<210> 51
   <211> 30
   <212> PRT
   <213> artificial sequence
<220>
   <223> peptide inhibiteur du entre Asf1 et H3-H4
<400> 51
   Gly Ala Met Gly Thr Leu Thr Pro Lys Glu Ala Glu Leu Ala Arg Arg
   1               5                   10                  15
   Ile Arg Gly Ala Gly Ala Ser Arg Thr Leu Asn Gly Phe Gly
               20                  25                  30
<210> 52
   <211> 39
   <212> PRT
   <213> Artificial
<220>
   <223> peptide inhibiteur du entre Asf1 et H3-H4
<400> 52
   Gly Ala Met Gly Leu Thr Ala Ala Glu His Ala Lys Arg Ser Thr Leu
   1               5                   10                  15
   Thr Pro Lys Glu Ala Gln Leu Ala Arg Arg Ile Arg Gly Ala Gly Gly
               20                  25                  30
   Val Thr Leu Asn Gly Phe Gly
           35
<210> 53
   <211> 35
   <212> PRT
   <213> artificial
<220>
   <223> peptide inhibiteur du entre Asf1 et H3-H4
<400> 53
   Leu Thr Ala Ala Glu His Ala Lys Arg Ser Thr Leu Thr Pro Lys Glu
   1               5                   10                  15
   Ala Glu Leu Ala Arg Arg Ile Arg Gly Ala Gly Gly Val Thr Leu Asn
               20                  25                  30
   Gly Phe Gly
           35
<210> 54
   <211> 36
   <212> PRT
   <213> artificial
<220>
   <223> peptide inhibiteur du entre Asf1 et H3-H4
<400> 54
   Leu Thr Ala Ala Glu His Ala Lys Arg Ser Thr Leu Thr Pro Lys Glu
   1               5                   10                  15
   Ala Gln Leu Ala Arg Arg Ile Glu Gly Ala Gly Ala Ser Val Thr Leu
               20                  25                  30
   Asn Gly Phe Gly
           35
<210> 55
   <211> 36
   <212> PRT
   <213> artificial
<220>
   <223> peptide inhibiteur du entre Asf1 et H3-H4
<400> 55
   Leu Thr Ala Ala Glu His Ala Lys Arg Ser Thr Leu Thr Pro Lys Glu
   1               5                   10                  15
   Ala Glu Leu Ala Arg Arg Ile Glu Gly Ala Gly Ala Ser Val Thr Leu
               20                  25                  30
   Asn Gly Phe Gly
           35
<210> 56
   <211> 29
   <212> PRT
   <213> artificial
<220>
   <223> peptide inhibiteur du entre Asf1 et H3-H4
<400> 56
   Gly Ala Met Gly Thr Arg Thr Pro Lys Glu Arg Arg Leu Ala Arg Arg
   1               5                   10                  15
   Ile Arg Gly Ala Gly Gly Arg Thr Leu Asn Gly Phe Gly
               20                  25
<210> 57
   <211> 29
   <212> PRT
   <213> artificial
<220>
   <223> peptide inhibiteur du entre Asf1 et H3-H4
<400> 57
   Gly Ala Met Gly Thr Arg Thr Pro Lys Glu Ala Arg Leu Ala Arg Arg
   1               5                   10                  15
   Ile Arg Gly Ala Gly Gly Arg Thr Leu Asn Gly Phe Gly
               20                  25
<210> 58
   <211> 39
   <212> PRT
   <213> artificial
<220>
   <223> peptide inhibiteur du entre Asf1 et H3-H4-peptide TAT-i42V
<400> 58
   Gly Arg Lys Lys Arg Arg Gln Arg Arg Arg Gly Ala Met Gly Thr Ile
   1               5                   10                  15
   Thr Pro Lys Glu Ala Gln Leu Ala Arg Arg Ile Arg Gly Ala Gly Gly
               20                  25                  30
   Val Thr Leu Asn Gly Phe Gly
           35
<210> 59
   <211> 44
   <212> PRT
   <213> artificial
<220>
   <223> peptide inhibiteur du entre Asf1 et H3-H4-peptide RRP5-i42V
<400> 59
   Arg Arg Pro Arg Arg Pro Arg Arg Pro Arg Arg Pro Arg Arg Pro Gly
   1               5                   10                  15
   Ala Met Gly Thr Ile Thr Pro Lys Glu Ala Gln Leu Ala Arg Arg Ile
               20                  25                  30
   Arg Gly Ala Gly Gly Val Thr Leu Asn Gly Phe Gly
           35                  40
<210> 60
   <211> 37
   <212> PRT
   <213> artificial
<220>
   <223> peptide RRP5-i1
<400> 60
   Arg Arg Pro Arg Arg Pro Arg Arg Pro Arg Arg Pro Arg Arg Pro Gly
   1               5                   10                  15
   Ala Met Gly Thr Ile Met Pro Lys Asp Ile Gln Leu Ala Arg Arg Ile
               20                  25                  30
   Arg Gly Gly Cys Ala
           35
<210> 61
   <211> 29
   <212> PRT
   <213> artificial
<220>
   <223> peptide inhibiteur du entre Asf1 et H3-H4
<400> 61
   Gly Ala Met Gly Thr Leu Thr Pro Lys Glu Ala Glu Leu Ala Arg Arg
   1               5                   10                  15
   Ile Arg Gly Ala Gly Gly Val Thr Tyr Asp Gly Phe Gly
               20                  25
<210> 62
   <211> 33
   <212> PRT
   <213> artificial
<220>
   <223> peptide inhibiteur du entre Asf1 et H3-H4
<400> 62
   Gly Ala Met Gly Thr Leu Thr Pro Lys Glu Ala Glu Leu Ala Arg Arg
   1 5 10 15
   Ile Arg Gly Ala Gly Gly Val Thr Leu Asn Gly Phe Gly Ala Ser Thr
   20 25 30
   Gly
<210> 63
   <211> 34
   <212> PRT
   <213> artificial
<220>
   <223> peptide inhibiteur du entre Asf1 et H3-H4
<400> 63
   Gly Ala Met Gly Thr Leu Thr Pro Lys Glu Ala Glu Leu Ala Arg Arg
   1 5 10 15
   Ile Arg Gly Ala Gly Gly Val Thr Leu Asn Gly Ala Asn Phe Val Ser
   20 25 30
   Thr Gly
<210> 64
   <211> 40
   <212> PRT
   <213> artificial
<220>
   <223> peptide inhibiteur du entre Asf1 et H3-H4
<400> 64
   Gly Ala Met Gly Arg Val Pro Pro Ala Val Arg Lys Leu Gly Asn Ser
   1 5 10 15
   Thr Glu Arg Lys Trp Ala Glu Leu Ala Arg Arg Ile Arg Gly Ala Gly
   20 25 30
   Gly Val Thr Leu Asn Gly Phe Gly
   35 40
<210> 65
   <211> 41
   <212> PRT
   <213> artificial
<220>
   <223> peptide inhibiteur du entre Asf1 et H3-H4
<400> 65
   Gly Ala Met Gly Arg Val Pro Pro Ala Val Arg Lys Leu Gly Asn Ala
   1               5                   10                  15
   Ser Thr Glu Arg Lys Trp Ala Glu Leu Ala Arg Arg Ile Arg Gly Ala
               20                  25                  30
   Gly Gly Val Thr Leu Asn Gly Phe Gly
           35                  40
<210> 66
   <211> 44
   <212> PRT
   <213> artificial
<220>
   <223> peptide inhibiteur du entre Asf1 et H3-H4 - peptide RRP5-i5
<400> 66
   Arg Arg Pro Arg Arg Pro Arg Arg Pro Arg Arg Pro Arg Arg Pro Ala
   1               5                   10                  15
   Ser Thr Glu Arg Lys Trp Ala Glu Leu Ala Arg Arg Ile Arg Gly Ala
               20                  25                  30
   Gly Gly Val Thr Leu Asn Gly Phe Gly Gly Cys Ala
           35                  40
<210> 67
   <211> 41
   <212> PRT
   <213> artificial
<220>
   <223> peptide inhibiteur du entre Asf1 et H3-H4
<400> 67
   Arg Arg Pro Arg Arg Pro Arg Arg Pro Arg Arg Pro Arg Arg Pro Ala
   1 5 10 15
   Ser Thr Glu Arg Lys Trp Ala Glu Leu Ala Arg Arg Ile Arg Gly Ala
   20 25 30
   Gly Gly Val Thr Leu Asn Gly Phe Gly
   35 40
<210> 68
   <211> 26
   <212> PRT
   <213> artificial
<220>
   <223> peptide i5 mut
<400> 68
   Ala Ser Thr Glu Arg Lys Trp Ala Glu Ala Ala Arg Arg Ala Arg Gly
   1 5 10 15
   Ala Gly Gly Val Thr Leu Asn Gly Ala Gly
   20 25
<210> 69
   <211> 44
   <212> PRT
   <213> artificial
<220>
   <223> peptide (RRP)5-i5 muté
<400> 69
   Arg Arg Pro Arg Arg Pro Arg Arg Pro Arg Arg Pro Arg Arg Pro Ala
   1 5 10 15
   Ser Thr Glu Arg Lys Trp Ala Glu Ala Ala Arg Arg Ala Arg Gly Ala
   20 25 30
   Gly Gly Val Thr Leu Asn Gly Ala Gly Gly Cys Ala
   35 40
<210> 70
   <211> 19
   <212> PRT
   <213> artificial
<220>
   <223> peptide i1
<400> 70
   Gly Ala Met Gly Thr Ile Met Pro Lys Asp Ile Gln Leu Ala Arg Arg
   1 5 10 15
   Ile Arg Gly
<210> 71
   <211> 19
   <212> PRT
   <213> artificial
<220>
   <223> Fragment H3 stabilisé
<400> 71
   Gly Ala Met Gly Thr Ile Thr Pro Lys Glu Ala Gln Leu Ala Arg Arg
   1 5 10 15
   Ile Arg Gly
<210> 72
   <211> 19
   <212> PRT
   <213> artificial
<220>
   <223> Fragment H3 stabilisé
<400> 72
   Gly Ala Met Gly Thr Leu Thr Pro Lys Glu Ala Glu Leu Ala Arg Arg
   1 5 10 15
   Ile Arg Gly
<210> 73
   <211> 27
   <212> PRT
   <213> artificial
<220>
   <223> peptide inhibiteur du entre Asf1 et H3-H4
<400> 73
   Gly Ala Met Gly Thr Leu Thr Pro Lys Glu Ala Glu Leu Ala Arg Arg
   1 5 10 15
   Ile Arg Gly Ala Arg Thr Leu Asn Gly Phe Gly
   20 25
<210> 74
   <211> 31
   <212> PRT
   <213> artificial
<220>
   <223> peptide inhibiteur du entre Asf1 et H3-H4
<400> 74
   Gly Ala Met Gly Thr Leu Thr Pro Lys Glu Ala Glu Leu Ala Arg Arg
   1               5                   10                  15
   Ile Arg Gly Ala Gly Ala Ser Gly Arg Thr Leu Asn Gly Phe Gly
               20                  25                  30
<210> 75
<211> 26
   <212> PRT
   <213> artificial sequence
<220>
   <223> Peptide i5_s3
<400> 75
   Ala Ser Thr Glu Glu Lys Trp Ala Arg Leu Ala Arg Arg Ile Arg Gly
   1               5                   10                  15
   Ala Gly Gly Val Thr Leu Asn Gly Phe Gly
               20                  25
<210> 76
   <211> 26
   <212> PRT
   <213> artificial sequence
<220>
   <223> Peptide i5_D
<400> 76
   Ala Ser Thr Glu Arg Lys Trp Ala Glu Leu Ala Arg Arg Ile Arg Gly
   1               5                   10                  15
   Ala Gly Gly Val Thr Leu Asp Gly Phe Gly
               20                  25
<210> 77
   <211> 26
   <212> PRT
   <213> artificial sequence
<220>
   <223> Peptide i5_loopR
<400> 77
   Ala Ser Thr Glu Arg Lys Trp Ala Glu Leu Ala Arg Arg Ile Arg Gly
   1               5                   10                  15
   Ala Gly Gly Arg Thr Leu Asn Gly Phe Gly
               20                  25
<210> 78
   <211> 26
   <212> PRT
   <213> artificial sequence
<220>
   <223> Peptide i5_loopA
<400> 78
   Ala Ser Thr Glu Arg Lys Trp Ala Glu Leu Ala Arg Arg Ile Arg Gly
   1               5                   10                  15
   Ala Gly Gly Ala Thr Leu Asn Gly Phe Gly
               20                  25
<210> 79
   <211> 26
   <212> PRT
   <213> artificial sequence
<220>
   <223> Peptide i5_Aloop
<400> 79
   Ala Ser Thr Glu Arg Lys Trp Ala Glu Leu Ala Arg Arg Ile Ala Gly
   1               5                   10                  15
   Ala Gly Gly Val Thr Leu Asn Gly Phe Gly
               20                  25
<210> 80
   <211> 26
   <212> PRT
   <213> artificial sequence
<220>
   <223> Peptide i6
<400> 80
   Ala Ser Thr Glu Glu Lys Trp Ala Arg Leu Ala Arg Arg Ile Ala Gly
   1               5                   10                  15
   Ala Gly Gly Val Thr Leu Asp Gly Phe Gly
               20                  25
<210> 81
   <211> 41
   <212> PRT
   <213> artificial sequence
<220>
   <223> Peptide RRP5-i6
<400> 81
   Arg Arg Pro Arg Arg Pro Arg Arg Pro Arg Arg Pro Arg Arg Pro Ala
   1               5                   10                  15
   Ser Thr Glu Glu Lys Trp Ala Arg Leu Ala Arg Arg Ile Ala Gly Ala
               20                  25                  30
   Gly Gly Val Thr Leu Asp Gly Phe Gly
                       35                  40
<210> 82
   <211> 27
   <212> PRT
   <213> artificial sequence
<220>
   <223> E1-B-E2 de formule (X)
<220>
   <221> MISC_FEATURE
   <222> (4)..(5)
   <223> Xaa est consécutivement X4 et X5 et est E ou R
<220>
   <221> MISC_FEATURE
   <222> (12)..(12)
   <223> Xaa est X8 étant E ou R
<220>
   <221> MISC_FEATURE
   <222> (15)..(15)
   <223> Xaa est X11 étant R ou A
<220>
   <221> MISC_FEATURE
   <222> (17)..(17)
   <223> Xaa est X12 étant A ou P
<220>
   <221> MISC_FEATURE
   <222> (19) .. (19)
   <223> Xaa est X13 étant absent ou sélectionné parmi les acides aminés G, A et S
<220>
   <221> MISC_FEATURE
   <222> (20)..(20)
   <223> Xaa est X14 étant absent ou S
<220>
   <221> MISC_FEATURE
   <222> (21) .. (21)
   <223> Xaa est X15 étant R, V ou A
<220>
   <221> MISC_FEATURE
   <222> (22)..(22)
   <223> Xaa est X16 étant T ou V
<220>
   <221> MISC_FEATURE
   <222> (24) .. (24)
   <223> Xaa est X18 étant Y, N ou D
<220>
   <221> MISC_FEATURE
   <222> (26)..(26)
   <223> Xaa est X19 étant F ou M
<220>
   <221> MISC_FEATURE
   <222> (27) .. (27)
   <223> Xaa est X20 étant G, Q ou N
<400> 82
   Ala Ser Thr Xaa Xaa Lys Trp Ala Glu Leu Ala Xaa Arg Ile Xaa Gly
   1               5                   10                  15
   Xaa Gly Xaa Xaa Xaa Xaa Leu Xaa Gly Xaa Xaa
               20                  25

## Description

La présente invention est relative à une nouvelle classe de molécules présentant un intérêt thérapeutique, en particulier dans le domaine de l'oncologie.

Pour protéger leur génome, les cellules ont développé plusieurs stratégies. D'une part, l'ADN est protégé des agressions oxydantes par une structure protéique compacte appelée chromatine. D'autre part, il existe des machineries de réparation et de signalisation qui prennent en charge les dommages de l'ADN pour restaurer un génome intact. Le chaperon d'histone Asf1 (Anti-Silencing Factor 1) se situe au carrefour de la réparation de l'ADN et des machineries moléculaires assurant le maintien de la chromatine.

Asf1 participe au dépôt des histones H3/H4 sur l'ADN lors de nombreux processus cellulaires. La délétion d'Asf1 dans différentes espèces (levure, humain, poulet, drosophile) ralentit la prolifération, sensibilise les cellules aux agents génotoxiques utilisés en chimiothérapie classique et empêche l'établissement de marques épigénétiques particulières, révélant ainsi le rôle crucial d'Asf1 dans l'assemblage de la chromatine, les réponses cellulaires aux dommages de l'ADN et le maintien de l'information épigénétique.

Des études très récentes ont montré que, dans des cellules humaines, l'inhibition d'Asf1 par des siRNA provoque le blocage en phase S des cellules. D'autre part, la surexpression de l'isoforme Asflb dans les cellules malignes constitue un marqueur particulièrement performant pour pronostiquer la gravité de cancers du sein (Corpet et al, 2011, Embo J. 30(3): 480-93). Ce dernier travail met en évidence, pour la première fois, l'implication du chaperon d'histones Asf1 dans le développement de cancers.

L'analyse structurale et fonctionnelle du chaperon d'histone Asf1 a permis de montrer que l'interaction entre Asf1 et les histones est requise pour l'ensemble de ses fonctions (Mousson, et al. 2005, PNAS USA, 2005. 102(17): 5975-5980; Agez et al. 2007, Structure, 2007. 15(2): 191-199; et Galvani et al. 2008, Molecular And Cellular Biology, 2008. 28(11): 3672-3685).

L'interaction entre Asf1 et les histones constitue donc une cible intéressante pour le développement de nouveaux agents anticancéreux. Des molécules capables d'inhiber cette interaction auront potentiellement des propriétés anticancéreuses non seulement par la perturbation de la prolifération, mais également de l'information épigénétique et par la sensibilisation les cellules aux agents anticancéreux classiques.

L'inhibition d'Asf1 par la méthode de siRNA est efficace sur des cellules en culture, mais il reste à l'heure actuelle certaines difficultés de la mettre en oeuvre comme médicament *in vivo.*

Les inventeurs fournissent par la présente invention une stratégie alternative basée sur une approche peptidique. Une stratégie classique pour concevoir des peptides inhibiteurs de l'interaction de deux protéines, notamment dans un complexe, consiste en la mise au point d'un peptide interagissant avec l'un des partenaires du complexe.

Les inventeurs, dans la présente invention, ont choisi de cibler plusieurs des partenaires de l'interaction avec Asf1, notamment à la fois l'histone H3 et l'histone H4. Ainsi, par la présente invention, les inventeurs fournissent des peptides inhibiteurs de la formation du complexe entre Asf1 et les histones H3-H4.

Un avantage de la présente invention vient du fait que la cible ne correspond pas à un enzyme ou un récepteur, comme c'est le plus souvent le cas, mais à une interaction protéine-protéine. En effet, il s'agit d'inhiber une interaction transitoire et labile entre le chaperon d'assemblage, Asf1, et une sous-unité du complexe visé, le nucléosome. Ainsi, un peptide agissant sur une interaction transitoire sera plus efficace que lorsque le peptide doit inhiber un complexe final stable. Cette stratégie peptidique présente en outre l'avantage d'une grande spécificité, prévenant ainsi des effets secondaires. Par ailleurs, le complexe ciblé, qui est vital, peut quand même se former spontanément en absence de protéine chaperon. On peut donc moduler la quantité de complexe formé et donc l'ampleur de l'action des molécules.

La présente invention concerne donc une molécule peptidique, capable d'inhiber la formation du complexe entre Asf1 et les histones H3-H4, comprenant ou consistant en les éléments E1-B-E2 (disposés dans le sens N-terminal vers C-terminal). E1 et E2 correspondant respectivement au peptide ciblant la région de liaison de l'histone H3 et celle de l'histone H4 et B désigne la boucle peptidique reliant ces deux éléments. Les éléments E1-B-E2 sont tels que définis ci-dessous :
a) E1 est un peptide de formule (I)

   X₁-X₂-X₃-X₄-X₅-K-X₆-X₇-X₈-L-X₉-X₁₀-R-I-X₁₁ (I)

   dans laquelle
   a1) soit (SEQ ID No 1)
      X₁ et X₂ sont absents ;
      X₃ est I, R ou L, de préférence I ou L ;
      X₄ est T ou M, de préférence T ;
      X₅ est P ;
      X₆ est E ou D, de préférence D ;
      X₇ est sélectionné parmi le groupe consistant en R, A, I et E, de préférence R, AetE;
      X₈ est sélectionné parmi le groupe consistant en R, Q et E ;
      X₉ est R ou A ;
      X₁₀ est R ; et
      X₁₁ est R ou E ;
   a2) soit (SEQ ID No 2)
      X₁, X₇ et X₉ sont A ;
      X₂ est S ;
      X₃ est T ;
      X₄, X₅, X₈ sont E ou R ;
      X₆ est W ;
      X₁₀ est R ; et
      X₁₁ est R ou A ;
b) B est un peptide de formule (II)

   G-X₁₂-G-X₁₃-X₁₄ (II)

   (SEQ ID No 3)
   dans laquelle
   X₁₂ est A ou P ;
   X₁₃ est absent ou sélectionné parmi les acides aminés G, A et S ;
   X₁₄ est absent ou S ;
   et
c) E2 est un peptide de formule (III)

   X₁₅-X₁₆-X₁₇-X₁₈-G-X₁₉-X₂₀ (III)

   (SEQ ID No 4)
   dans laquelle
   X₁₅ est R, V ou A ;
   X₁₆ est T ou V ;
   X₁₇ est L ;
   X₁₈ est Y, N ou D ;
   X₁₉ est F ou M ; et
   X₂₀ est sélectionné par le groupe consistant en G, Q et N.

Plus particulièrement, la molécule peptidique peut comprendre également les éléments E1-B-E2, dans laquelle
soit
X₁ et X₂ sont absents ;
X₃ est I, R ou L, de préférence I ou L ;
X₄ est T ou M, de préférence T ;
X₅ est P ;
X₆ est E ou D, de préférence D ;
X₇ est sélectionné parmi le groupe consistant en R, A, I et E, de préférence R, AetE;
X₈ est sélectionné parmi le groupe consistant en R, Q et E ;
X₉ est R ou A ;
X₁₀ est R ; et
X₁₁ est R ou E ;
soit
X₁, X₇ et X₉ sont A ;
X₂ est S ;
X₃ est T ;
X₄ est E ou R ;
X₆ est W ;
X₈ est E ; et
X₅, X₁₀ et X₁₁ sont R ;
et
X₁₂ est A ou P ;
X₁₃ est absent ou sélectionné parmi les acides aminés G, A et S ;
X₁₄ est absent ou S ;
X₁₅ est R ou V ;
X₁₆ est T ou V ;
X₁₇ est L ;
X₁₈ est Y, N ou D ;
X₁₉ est F ou M, de manière encore plus préférée est F ; et
X₂₀ est sélectionné par le groupe consistant en G, Q et N.

Dans un mode de réalisation tout particulièrement préféré, la présente invention concerne une molécule peptidique, capable d'inhiber la formation du complexe entre Asf1 et les histones H3-H4, comprenant ou consistant en les éléments E1-B-E2 telle que définis ci-dessus, dans laquelle les éléments E1-B-E2 présentent l'une des formules suivantes

X₃-X₄-P-K-X₆-X₇-X₈-L-X₉-R-R-I-X₁₁-G-X₁₂-G-X₁₃-X₁₄-X₁₅-X₁₆-L-X₁₈-G-X₁₉-X₂₀ (VIII)

(SEQ ID No 11) dans laquelle
X₃ est I, R ou L, de préférence I ou L ;
X₄ est T ou M, de préférence T ;
X₆ est E ou D, de préférence D ;
X₇ est sélectionné parmi le groupe consistant en R, A, I et E, de préférence parmi R, A etE;
X₈ est sélectionné parmi le groupe consistant en R, Q et E ;
X₉ est R ou A ;
X₁₁ est R ou E ;
X₁₂ est A ou P ;
X₁₃ est absent ou sélectionné parmi les acides aminés G, A et S ;
X₁₄ est absent ou S ;
X₁₅ est R ou V ;
X₁₆ est T ou V ;
X₁₈ est Y, N ou D ;
X₁₉ est sélectionné par le groupe consistant en F, M, A et Q, de préférence F ou M, de manière encore plus préféré F ; et
X₂₀ est sélectionné par le groupe consistant en G, Q et N ;
ou

A-S-T-X₄-R-K-W-A-E-L-A-R-R-I-R-G-X₁₂-G-X₁₃-X₁₄-X₁₅-X₁₆-L-X₁₈-G-X₁₉-X₂₀ (IX) (SEQ ID No 12)

dans laquelle
X₄ est E ou R ;
X₁₂ est A ou P ;
X₁₃ est absent ou sélectionné parmi les acides aminés G, A et S ;
X₁₄ est absent ou S ;
X₁₅ est R ou V ;
X₁₆ est T ou V ;
X₁₈ est Y, N ou D ;
X₁₉ est sélectionné par le groupe consistant en F, M, A et Q, de préférence F ou M, de manière encore plus préféré F ; et
X₂₀ est sélectionné par le groupe consistant en G, Q et N,
ou

A-S-T-X₄-X₅-K-W-A-E-L-A-X₈-R-I-X₁₁-G-X₁₂-G-X₁₃-X₁₄-X₁₅-X₁₆-L-X₁₈-G-X₁₉-X₂₀ (XI)

(SEQ ID No 82) dans laquelle
X₄, X₅, X₈ sont E ou R ;
X₁₁ est R ou A ;
X₁₂ est A ou P ;
X₁₃ est absent ou sélectionné parmi les acides aminés G, A et S ;
X₁₄ est absent ou S ;
X₁₅ est R, V ou A ;
X₁₆ est T ou V ;
X₁₈ est Y, N ou D ;
X₁₉ est F ou M, de préférence F ; et
X₂₀ est sélectionné par le groupe consistant en G, Q et N.

Dans un mode de réalisation particulier, la présente invention concerne une molécule peptidique comprenant ou consistant en les éléments E1-B-E2 telle que définis ci-dessus, dans laquelle les éléments E1-B-E2 présentent l'une des séquences suivantes
IMPKDIQLARRIRGAGGRTLYGFG (SEQ ID No 13)
IMPKDIQLARRIRGAGASRTLYGFG (SEQ ID No 14)
ITPKEAQLARRIRGAGGRTLNGFG (SEQ ID No 15)
ITPKEAQLARRIRGAGASRTLNGFG (SEQ ID No 16)
LTPKEAELARRIRGAGGRTLNGFG (SEQ ID No 17)
LTPKEAELARRIRGAGASRTLNGFG (SEQ ID No 18)
ITPKEAQLARRIEGAGASVTLNGFG (SEQ ID No 19)
LTPKEAELARRIEGAGASVTLNGFG (SEQ ID No 20)
ITPKEEQLRRRIEGAGASVTLNGFG (SEQ ID No 21)
ITPKEAQLARRIRGAGGVTLNGFG (SEQ ID No 22)
LTPKEAELARRIRGAGGVTLNGFG (SEQ ID No 23)
LTPKEAELARRIRGAGRTLNGFG (SEQ ID No 24)
LTPKEAELARRIRGAGGRTLNGFG (SEQ ID No 25)
LTPKEAELARRIRGAGASRTLNGFG (SEQ ID No 26)
RTPKERRLARRIRGAGGRTLNGFG (SEQ ID No 27)
RTPKEARLARRIRGAGGRTLNGFG (SEQ ID No 28)
LTPKEAELARRIRGAGGVTYDGFG (SEQ ID No 29)
LTPKEAELARRIRGAGGVTLNGAN (SEQ ID No 30)
STERKWAELARRIRGAGGVTLNGFG (SEQ ID No 31)
ASTERKWAELARRIRGAGGVTLNGFG (SEQ ID No 32)
ASTRRKWAELARRIRGAGGVTLNGFG (SEQ ID No 33),
ASTEEKWARLARRIRGAGGVTLNGFG (SEQ ID No 75)
ASTERKWAELARRIRGAGGVTLDGFG (SEQ ID No 76)
ASTERKWAELARRIRGAGGRTLNGFG (SEQ ID No 77)
ASTERKWAELARRIRGAGGATLNGFG (SEQ ID No 78)
ASTERKWAELARRIAGAGGVTLNGFG (SEQ ID No 79)
ASTEEKWARLARRIAGAGGVTLDGFG (SEQ ID No 80)
ainsi qu'une séquence présentant 90 ou 95 % d'identité avec celles-ci.

Par « pourcentage d'identité » entre deux séquences d'acides aminés, on entend, dans le cadre de la présente invention, un pourcentage de résidus d'acides aminés identiques entre les deux séquences comparées, ce pourcentage étant obtenu après mise en oeuvre du meilleur alignement (alignement optimum) entre les deux séquences. L'homme du métier connait différentes techniques permettant d'obtenir un tel pourcentage d'identité et impliquant des algorithmes d'homologie ou des programmes d'ordinateur tels que l'algorithme d'alignement global de Néedleman-Wunsch en prenant en compte les éventuels "gaps", le programme BLAST de NCBI ou l'outil d'alignement "EMBOSS Pairwise Alignment Algorithms" disponible notamment sur le site internet de l'EMBL-EBI. Notamment, le pourcentage d'identité par rapport à la séquence de référence sera calculé en divisant (i) le nombre total de résidus identiques alignés entre les deux séquences par (ii) le nombre total de résidus contenus dans la séquence de référence, puis en multipliant par 100 le quotient obtenu.

De préférence, la molécule peptidique selon la présente invention comprend en outre un élément facilitant la pénétration cellulaire de la molécule. En particulier, cet élément est un peptide facilitant la pénétration cellulaire de la molécule (élément CPP). Ces peptides sont bien connus de l'homme du métier (par exemple, Vivès et al, Biochimic et Biophysica Acta, 2008, 1786, 126-138). Par exemple et à titre non limitatif, le peptide facilitant la pénétration cellulaire peut être choisi par le groupe consistant en un peptide de Tat, notamment RKKRRQRRR (SEQ ID No 34), un peptide d'antennapedia ou penetratin, notamment RQIKIWFQNRRMKWKK (SEQ ID No 35), et un peptide riche en arginine et en lysine, de préférence riche en arginine, notamment un peptide comprenant au moins 9 résidus d'arginine tel qu'un poly-arginine RRRRRRRRR (SEQ ID No 36) ou RRPRRPRRPRRPRRP (SEQ ID No 37).

Ce peptide facilitant la pénétration cellulaire de la molécule peut être présent du coté N-terminal ou C-terminal des éléments E1-B-E2, de préférence du coté N-terminal de ces éléments. Il peut soit être lié directement aux éléments E1-B-E2, soit y être lié par l'intermédiaire d'un peptide.

Facultativement, la molécule peptidique peut comprendre en outre d'autres séquences peptidiques, du coté N-terminal ou C-terminal des éléments E1-B-E2 ou/et entre ces éléments et le peptide facilitant la pénétration cellulaire et/ou à l'extrémité du peptide facilitant la pénétration cellulaire.

Dans un mode de réalisation très particulier, la molécule peptide comprend ou consiste en une séquence choisie parmi le groupe consistant en
GAMGTIMPKDIQLARRIRGAGGRTLYGFG (SEQ ID No 38)
GAMGTIMPKDIQLARRIRGAGASRTLYGFG (SEQ ID No 39)
GAMGTITPKEAQLARRIRGAGGRTLNGFG (SEQ ID No 40)
GAMGTITPKEAQLARRIRGAGASRTLNGFG (SEQ ID No 41)
GAMGTLTPKEAELARRIRGAGGRTLNGFG (SEQ ID No 42)
GAMGTLTPKEAELARRIRGAGASRTLNGFG (SEQ ID No 43)
GAMGTITPKEAQLARRIEGAGASVTLNGFG (SEQ ID No 44)
GAMGTLTPKEAELARRIEGAGASVTLNGFG (SEQ ID No 45)
GAMGTITPKEEQLRRRIEGAGASVTLNGFG (SEQ ID No 46)
GAMGTITPKEAQLARRIRGAGGVTLNGFG (SEQ ID No 47)
GAMGTLTPKEAELARRIRGAGGVTLNGFG (SEQ ID No 48)
GAMGTLTPKEAELARRIRGAGRTLNGFG (SEQ ID No 49)
GAMGTLTPKEAELARRIRGAGASRTLNGFG (SEQ ID No 51)
GAMGLTAAEHAKRSTLTPKEAQLARRIRGAGGVTLNGFG (SEQ ID No 52)
LTAAEHAKRSTLTPKEAELARRIRGAGGVTLNGFG (SEQ ID No 53)
LTAAEHAKRSTLTPKEAQLARRIEGAGASVTLNGFG (SEQ ID No 54)
LTAAEHAKRSTLTPKEAELARRIEGAGASVTLNGFG (SEQ ID No 55)
GAMGTRTPKERRLARRIRGAGGRTLNGFG (SEQ ID No 56)
GAMGTRTPKEARLARRIRGAGGRTLNGFG (SEQ ID No 57)
GRKKRRQRRRGAMGTITPKEAQLARRIRGAGGVTLNGFG (SEQ ID No 58)
RRPRRPRRPRRPRRPGAMGTITPKEAQLARRIRGAGGVTLNGFG (SEQ ID No 59)
GAMGTLTPKEAELARRIRGAGGVTYDGFG (SEQ ID No 61)
GAMGTLTPKEAELARRIRGAGGVTLNGFGASTG (SEQ ID No 62)
GAMGTLTPKEAELARRIRGAGGVTLNGANFVSTG (SEQ ID No 63)
GAMGRVPPAVRKLGNSTERKWAELARRIRGAGGVTLNGFG (SEQ ID No 64)
GAMGRVPPAVRKLGNASTERKWAELARRIRGAGGVTLNGFG (SEQ ID No 65)
ASTERKWAELARRIRGAGGVTLNGFG (SEQ ID No 32)
ASTRRKWAELARRIRGAGGVTLNGFG (SEQ ID No 33),
ASTEEKWARLARRIRGAGGVTLNGFG (SEQ ID No 75)
ASTERKWAELARRIRGAGGVTLDGFG (SEQ ID No 76)
ASTERKWAELARRIRGAGGRTLNGFG (SEQ ID No 77)
ASTERKWAELARRIRGAGGATLNGFG (SEQ ID No 78)
ASTERKWAELARRIAGAGGVTLNGFG (SEQ ID No 79)
ASTEEKWARLARRIAGAGGVTLDGFG (SEQ ID No 80)
RRPRRPRRPRRPRRPASTERKWAELARRIRGAGGVTLNGFGGCA (SEQ ID No 66)
RRPRRPRRPRRPRRPASTERKWAELARRIRGAGGVTLNGFG (SEQ ID No 67)
RRPRRPRRPRRPRRPGAMGTITPKEAQLARRIRGAGGVTLNGFGCA (SEQ ID No 50)
RRPRRPRRPRRPRRPASTEEKWARLARRIAGAGGVTLDGFG (SEQ ID No 81)
ainsi qu'une séquence présentant 90 ou 95 % d'identité avec celles-ci.

Dans un mode de réalisation tout particulièrement préféré, la molécule peptide comprend ou consiste en une séquence choisie parmi le groupe consistant en
RRPRRPRRPRRPRRPGAMGTITPKEAQLARRIRGAGGVTLNGFGCA (SEQ ID No 50)
RRPRRPRRPRRPRRPGAMGTITPKEAQLARRIRGAGGVTLNGFG (SEQ ID No 59)
RRPRRPRRPRRPRRPASTERKWAELARRIRGAGGVTLNGFGGCA (SEQ ID No 66)
RRPRRPRRPRRPRRPASTERKWAELARRIRGAGGVTLNGFG (SEQ ID No 67) et
RRPRRPRRPRRPRRPASTEEKWARLARRIAGAGGVTLDGFG (SEQ ID No 81).

La molécule peptidique selon la présente invention peut comprendre des acides aminés non-naturels. Par « acide aminé non-naturel » est entendu un analogue ou dérivé d'un acide aminé naturel. Par exemple, un acide aminé non-naturel peut présenter une chaine latérale allongée, plus courte, ou variante présentant des groupements fonctionnels appropriés.

Bien entendu, les isomères L et D des acides aminés sont envisagés. En effet, les isomères D ne sont pas sensibles aux protéases et la présente invention comprend également des molécules comprenant uniquement ou essentiellement des D acides aminés. Dans un mode particulier, les acides aminés L sont préférés.

Les séquences peptidiques définies dans le présent document sont représentées avec le symbole en une lettre tel qu'indiqué ci-dessous:

| | | |
|---|---|---|
| A | Ala | (alanine) |
| R | Arg | (arginine) |
| N | Asn | (asparagine) |
| D | Asp | (acide aspartique) |
| C | Cys | (cystéine) |
| Q | Gln | (glutamine) |
| E | Glu | (acide glutamique) |
| G | Gly | (glycine) |
| H | His | (histidine) |
| I | Ile | (isoleucine) |
| L | Leu | (leucine) |
| K | Lys | (lysine) |
| M | Met | (méthionine) |
| F | Phe | (phénylalanine) |
| P | Pro | (proline) |
| S | Ser | (sérine) |
| T | Thr | (thréonine) |
| W | Trp | (tryptophane) |
| Y | Tyr | (tyrosine) |
| V | Val | (valine) |

Par ailleurs, les ou des liaisons peptidiques de la molécule peptidique selon la présente invention peuvent être modifiées pour les rendre résistantes à la protéolyse. Par exemple, au moins une liaison peptidique (-CO-NH-) peut être remplacée par une liaison divalente choisie parmi (-CH₂-NH-), (-NH-CO-), (-CH₂-O-), (-CH₂-S-), (-CH₂-CH₂-), (-CO-CH₂-), (-CHOH-CH₂-), (-N=N-), et (-CH=CH-). Facultativement, toutes les liaisons peptidiques peuvent être remplacées.

La molécule peptidique peut comprendre soit une extrémité C terminale carboxylique (-COO⁻) ou amidée (-CONH₂). Le peptide peut également être facultativement modifié à son extrémité N-terminale, par exemple par un radical acétyle.

En outre, la molécule peptidique selon la présente invention peut être modifiée pour la rendre plus stable, et notamment plus résistante aux protéases. Ainsi, la molécule peut porter des groupements PEG (polyéthylèneglycol). Les procédés de PEGylation sont bien connus de l'homme du métier (Olson et al, 2009, Integrative Biology, 1(5-6): p. 382-393).

Par « acide aminé favorable à une structure en hélice alpha » est entendu notamment un acide aminé qui est adapté ou favorable à l'adoption d'une structure secondaire en hélice alpha. En particulier, cela exclut les acides aminés défavorables à cette structure secondaire comme T, V, P et I. Ainsi, ce résidu pourra être sélectionné dans la liste consistant en A, R, D, N, C, G, Q, E, H, L, K, M, F, S, W et Y, de préférence parmi A, R, D, N, C, Q, E, H, L, K, M, F, S, et W, de manière encore plus préférée une combinaison d'acides aminés peut être sélectionnée avec l'aide de logiciels de prédiction de stabilité des structures hélicoïdales bien connues de l'homme du métier, tel que AGADIR.

De préférence, la molécule peptidique a une taille maximale de 200, 150 ou 100 acides aminés.

La présente description décrit également un acide nucléique codant pour la molécule peptidique selon l'invention ainsi qu'une cassette d'expression ou qu'un vecteur d'expression comprenant cet acide nucléique et capable d'exprimer et de produire la dite molécule dans des conditions appropriées. Ainsi, la molécule peptidique peut être produite par génie génétique. Alternativement, la molécule peut également être produite par une méthode de synthèse peptique, bien connue de l'homme du métier.

La présente invention concerne en outre une molécule peptidique selon la présente invention en tant que médicament. De préférence, la présente invention concerne une molécule peptidique selon la présente invention en tant qu'agent anticancéreux ou qu'agent sensibilisant les cellules cancéreuses aux agents anticancéreux.

La présente invention concerne une composition pharmaceutique comprenant une molécule peptidique selon la présente invention et un support pharmaceutiquement acceptable. Facultativement, la composition peut comprendre en outre un autre agent thérapeutique, en particulier un agent anticancéreux. De préférence, la composition est destinée au traitement du cancer.

La présente invention concerne une molécule peptidique selon la présente invention pour utilisation dans le traitement ou la prévention du cancer, facultativement en combinaison avec un autre agent thérapeutique, en particulier un agent anticancéreux, et/ou avec la radiothérapie. Ainsi, la présente invention concerne également un produit ou kit comprenant une molécule peptidique selon la présente invention et un agent anticancéreux en tant que préparation combinée destinée à une utilisation simultanée, séparée ou séquentielle, en particulier pour une utilisation dans le traitement du cancer.

En outre, la présente invention concerne l'utilisation d'une molécule peptidique selon la présente invention pour la préparation d'un médicament destiné au traitement ou la prévention du cancer, facultativement en combinaison avec un autre agent thérapeutique, en particulier un agent anticancéreux, et/ou avec la radiothérapie.

Enfin, la présente description décrit une méthode de traitement d'un cancer chez un patient, comprenant l'administration au patient d'une quantité thérapeutiquement efficace d'une molécule peptidique selon la présente invention. Facultativement, la méthode peut comprendre en outre l'administration d'un autre agent anticancéreux et/ou le traitement par radiothérapie.

Dans le contexte de la présente invention, le terme « traitement » ou « traiter » inclut le traitement préventif, curatif, palliatif, ainsi que la prise en charge des patients (réduction de la souffrance, amélioration de la durée de vie, ralentissement de la progression de la maladie, réduction de la croissance tumorale, diminution de la taille des tumeurs, la prévention ou la diminution des métastases et des rechutes etc.).

Les cancers incluent les cancers à tumeurs solides ou les cancers hématopoïétiques. Les cancers peuvent être choisis, de manière non-exhaustive, parmi les cancers du sein, les ostéocarcinomes, les cancers de la peau, des ovaires, des poumons, de l'utérus, du col de l'utérus, du vagin, de la prostate, des testicules, de la tyroïde, du système lymphatique, du sang, du foie, du pancréas, du rein, de la vessie, du cerveau ou du colon, les cancers gastriques.

La présente demande concerne la combinaison de la molécule selon la présente invention avec un autre agent anticancéreux qui peut être choisi par la chimiothérapie, la radiothérapie, l'hormonothérapie, l'immunothérapie, et la thérapie génique.

Les traitements et médicaments de l'invention sont tout particulièrement destinés aux humains.

Les molécules ou compositions selon l'invention peuvent être administrés de différentes manières et sous différentes formes. Ainsi, ils peuvent être administrés de manière systémique, par voie orale, par inhalation ou par injection, comme par exemple par voie intraveineuse, intra-musculaire, sous-cutanée, trans-dermique, intra-artérielle, etc., les voies intraveineuse, intra-musculaire, sous-cutanée, orale et par inhalation étant préférées. Pour les injections, les molécules sont généralement conditionnées sous forme de suspensions liquides, qui peuvent être injectées au moyen de seringues ou de perfusions, par exemple. A cet égard, les molécules sont généralement dissoutes dans des solutions salines, physiologiques, isotoniques, tamponnées, etc., compatibles avec un usage pharmaceutique et connues de l'homme du métier. Ainsi, les compositions peuvent contenir un ou plusieurs agents, supports ou véhicules choisis parmi les dispersants, solubilisants, stabilisants, conservateurs, etc. Des agents ou véhicules utilisables dans des formulations liquides et/ou injectables sont notamment la méthylcellulose, l'hydroxyméthylcellulose, la carboxyméthylcellulose, le polysorbate 80, le mannitol, la gélatine, le lactose, des huiles végétales, l'acacia, etc.

Les molécules peuvent également être administrées sous forme de gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Par « quantité efficace » est entendue la quantité qui est nécessaire pour prévenir, abolir ou diminuer les effets délétères du cancer. Il est entendu que le débit et/ou la dose injectée peuvent être adaptés par l'homme du métier en fonction du patient, de la pathologie concernée, du mode d'administration, etc. Typiquement, les molécules sont administrées à des doses pouvant varier entre 1 µg et 1000 mg/kg de poids corporel, plus généralement de 10 µg à 100 mg/kg, typiquement entre 1 mg et 20 mg/kg. En outre, des injections répétées peuvent être réalisées, le cas échéant. D'autre part, pour des traitements chroniques, des systèmes retard ou prolongés peuvent être avantageux.

Les exemples illustrent la présente invention et ne sont pas destinés à limiter celle-ci.

### Description des figures

**Figure 1****:** Courbes ITC de l'interaction Asf1 1-156 (30µM en A, 10µM en B et C) avec le peptide i1 (A), i4(B) et i5 (C). La concentration de i1 utilisée pour cette expérience est de 0,5mM, et 0,16mM pour i4 et i5.
**Figure 2****:** Structure RMN (A) et cristallographique (B et C) d'Asf1 en interaction avec le peptide i1 (A) i4 (B) et i5 (C).
**Figure 3****:** Analyse par Western Blot du test de compétition sur colonne GST de l'interaction Asf1-H3-H4 par i5 (ou i5mut), révélé par un anticorps anti-Asf1 (hAsf1a) et anti-Histone H3.
**Figure 4:** Figure 4A: Visualisation par microscopie de Fluorescence (en haut) et optique (en bas) du peptide i5, i5mut et des histones H3 entières étiquetés avec la GFP (Green fluorescent protein) dans des cellules humaines U2OS, Figure 4B: co-immunoprécipitation de Asf1 avec l'histone H3 entière, mutée (H3-R3A) et avec les peptide i5 et i5mut, révélée par un anticorps anti-Asf1(hAsf1a) et anti-GFP. L'astérisque indique la présence de produits de dégradation (GFP seule)
**Figure 5****:** Analyse de la pénétration des peptides (portant un FITC) dans les cellules U2OS par cytométrie de flux
**Figure 6** **:** Résultats de toxicité à 24h sur des cellules U2OS transduites avec différents peptides sans peptide (T WT) sans peptide en présence de Pyrène Buryrate (T PyBu), en présence de Pyrène Buryrate et le peptide indiqué à la concentration indiquée.
**Figure 7** **:** Perturbation de la progression du cycle cellulaire par l'action du peptide i5 ou i5mut.
**Figure 8** : Analyse par Western Blot du test de compétition sur colonne GST de l'interaction Asf1-H3-H4 par i5, i5mut, i6, révélé par un anticorps anti-GST (GST-Asf1a) et anti-Histone H3. E0 : pas de peptide, E5 : 5 µM, E10 : 10 µM, E50 : 50 µM, E100 : 100 µM.
**Figure 9** **:** Mesure de la toxicité cellulaire (test MTT) des peptides i5mut, i5 et i6 48H après transfection dans des cellules U2OS sous forme de plasmide codant pour une protéine de fusion GFP-peptide.
**Figure 10** **:** Mesure de la toxicité cellulaire (test MTT) des peptides i5mut, i5, et i6 48H après transfection dans des cellules Hela sous forme de peptide fusionné avec la séquence (RRP)₅

### EXEMPLES

### Résultats

### Stratégie de conception

La stratégie que les inventeurs ont adoptée a consisté à mimer non pas un partenaire, mais plusieurs partenaires et à optimiser par un lien le plus rigide possible pour le couplage des épitopes de liaison de chacun des partenaires afin d'augmenter l'affinité de la molécule chimérique ainsi générée. Ce design rationnel a été effectué *in silico,* puis validé par plusieurs méthodes biophysiques complémentaires. Enfin, la caractérisation de l'activité biologique a été effectuée sur des cellules humaines en culture.

### Optimisation du design du peptide inhibiteur de l'interaction H3H4:

L'objectif de l'invention est le design d'un peptide inhibiteur de l'interaction entre le chaperon Asf1 et les histones H3 et H4. Pour ce faire, les inventeurs ont couplé les épitopes de liaison des histones H3 et H4 à Asf1 entre eux. Pour cela, l'hélice α3 de l'histone H3 (peptide i1, SEQ ID No 70) a été reliée avec le brin β C-terminal de l'histone H4 par une boucle peptidique, dont la longueur et la composition ont été optimisées. Plusieurs acides aminés des épitopes de H3 et H4 ont été modifiés, afin de stabiliser la forme liée du peptide, de le rendre plus soluble et plus résistante aux protéases afin d'améliorer sa durée de vie dans les cellules humaines. Les inventeurs ont alors obtenu une série de peptides notés i4, i4_1, i4_2, i4V, i42V. Enfin, certaines modifications de la séquence ont permis d'optimiser l'interaction du peptide avec Asf1 (peptide i5, SEQ ID No 32).

### Schéma de conception du peptide

**Peptide de départ : fragment de l'histone H3**

| | | |
|---|---|---|
| SEQ ID No 70 (i1) | GAMGTIMPKDIQLARRIRG | Kd=8,7 µM |

la présence des résidus GAMG résultant du vecteur de clonage utilisé.

**Stabilisation du peptide**

| | | |
|---|---|---|
| SEQ ID No 71 | GAMGTITPKEAQLARRIRG | Kd=1,4 µM |
| SEQ ID No 72 | GAMGTLTPKEAELARRIRG | Kd=2,23 µM |

**Peptides couplant le domaine H3 au domaine H4 via une boucle**

| | | |
|---|---|---|
| SEQ ID No 38 (i4_1) | GAMGTIMPKDIQLARRIRGAGGRTLYGFG | Kd=0,56 µM |
| SEQ ID No 39 | GAMGTIMPKDIQLARRIRGAGASRTLYGFG | Kd=0,35 µM |
| SEQ ID No 40 (i4_2) | GAMGTITPKEAQLARRIRGAGGRTLNGFG | Kd=0,22 µM |
| SEQ ID No 41 | GAMGTITPKEAQLARRIRGAGASRTLNGFG | Kd=0,80 µM |
| SEQ ID No 42 (i4) | GAMGTLTPKEAELARRIRGAGGRTLNGFG | Kd=0,18 µM |
| SEQ ID No 43 | GAMGTLTPKEAELARRIRGAGASRTLNGFG | Kd=0,35 µM |

**Peptides plus résistants aux protéases**

| | | |
|---|---|---|
| SEQ ID No 44 | GAMGTITPKEAQLARRIEGAGASVTLNGFG | Kd=0,66 µM |
| SEQ ID No 45 | GAMGTLTPKEAELARRIEGAGASVTLNGFG | Kd=2,00 µM |
| SEQ ID No 46 | GAMGTITPKEEQLRRRIEGAGASVTLNGFG | Kd=0,42 µM |
| SEQ ID No 47 (i42V) | GAMGTITPKEAQLARRIRGAGGVTLNGFG | Kd=0,35 µM |
| SEQ ID No 48 (i4V) | GAMGTLTPKEAELARRIRGAGGVTLNGFG | Kd=0,06 µM |

**Test sur les longueurs de boucle**

| | | |
|---|---|---|
| SEQ ID No 73 | GAMGTLTPKEAELARRIRGARTLNGFG | Kd=3,89 µM |
| SEQ ID No 49 | GAMGTLTPKEAELARRIRGAGRTLNGFG | Kd=0,95 µM |
| SEQ ID No 42 | GAMGTLTPKEAELARRIRGAGGRTLNGFG | Kd=0,18 µM |
| SEQ ID No 51 | GAMGTLTPKEAELARRIRGAGASRTLNGFG | Kd=0,35 µM |
| SEQ ID No 74 | GAMGTLTPKEAELARRIRGAGASGRTLNGFG | Kd=1,89 µM |

**Test sur l'allongement en N terminal**

| | | |
|---|---|---|
| SEQ ID No 52 | GAMGLTAAEHAKRSTLTPKEAQLARRIRGAGGVTLNGFG | Kd=0,03 µM |
| SEQ ID No 53 | LTAAEHAKRSTLTPKEAELARRIRGAGGVTLNGFG | Kd=0,06 µM |
| SEQ ID No 54 | LTAAEHAKRSTLTPKEAQLARRIEGAGASVTLNGFG | Kd=0,12 µM |
| SEQ ID No 55 | LTAAEHAKRSTLTPKEAELARRIEGAGASVTLNGFG | Kd=0,88 µM |

**Test pour transformer le peptide en peptide pénétrant les cellules (CPP)**

| | | |
|---|---|---|
| SEQ ID No 56 | GAMGTRTPKERRLARRIRGAGGRTLNGFG | Kd=0,03 µM |
| SEQ ID No 57 | GAMGTRTPKEARLARRIRGAGGRTLNGFG | Kd=0,26 µM |

**Test pour l'ajout de peptides pénétrant les cellules (CPP)**

| | | |
|---|---|---|
| SEQ ID No 58 (TAT-i42V) | GRKKRRQRRRGAMGTITPKEAQLARRIRGAGGVTLNGFG | Kd=0,62 µM |
| SEQ ID No 59 (RRP5-i42V) | RRPRRPRRPRRPRRPGAMGTITPKEAQLARRIRGAGGVTLNGFG | Kd=0,59 µM |
| SEQ ID No 60 (RRP5-i1) | RPRRPRRPRRPRRPGAMGTIMPKDIQLARRIRGGCA | Kd=0,46 µM |
| SEQ ID No 50 (RRP5-i42V-CA) | RRPRRPRRPRRPRRPGAMGTITPKEAQLARRIRGAGGVTLNGFGCA | Kd=0,42 µM |

**Test sur l'allongement en C terminal**

| | | |
|---|---|---|
| SEQ ID No 61 | GAMGTLTPKEAELARRIRGAGGVTYDGFG | Kd=0,10 µM |
| SEQ ID No 62 | GAMGTLTPKEAELARRIRGAGGVTLNGFGASTG | Kd=0,32 µM |
| SEQ ID No 63 | GAMGTLTPKEAELARRIRGAGGVTLNGANFVSTG | Kd=1,68 µM |

**Optimisation de la liaison avec Asf1**

| | | |
|---|---|---|
| SEQ ID No 64 | GAMGRVPPAVRKLGNSTERKWAELARRIRGAGGVTLNGFG | Kd=0,08 µM |
| SEQ ID No 65 | GAMGRVPPAVRKLGNASTERKWAELARRIRGAGGVTLNGFG | Kd=0,06 µM |
| SEQ ID No 32 (i5) | ASTERKWAELARRIRGAGGVTLNGFG | Kd=0,04 µM |
| SEQ ID No 33 | ASTRRKWAELARRIRGAGGVTLNGFG | Kd=1,33 µM |
| SEQ ID No 66 (RRP5-i5) | RRPRRPRRPRRPRRPASTERKWAELARRIRGAGGVTLNGFGGCA | Kd=0,67 µM |
| SEQ ID No 75 (i5_s3) | ASTEEKWARLARRIRGAGGVTLNGFG | Kd=0,02 µM |
| SEQ ID No 76 (i5_D) | ASTERKWAELARRIRGAGGVTLDGFG | Kd=0,009µM |
| SEQ ID No 77 (i5_loopR) | ASTERKWAELARRIRGAGGRTLNGFG | Kd= 0,029µM |
| SEQ ID No 78 (i5_loopA) | ASTERKWAELARRIRGAGGATLNGFG | Kd=0,063 µM |
| SEQ ID No 79 (i5_Aloop) | ASTERKWAELARRIAGAGGVTLNGFG | Kd=0,027 µM |
| SEQ ID No 80 (i6) | ASTEEKWARLARRIAGAGGVTLDGFG | Kd=0,003 µM |
| SEQ ID No 81 (RRP5-i6) | RRPRRPRRPRRPRRPASTEEKWARLARRIAGAGGVTLDGFG | |

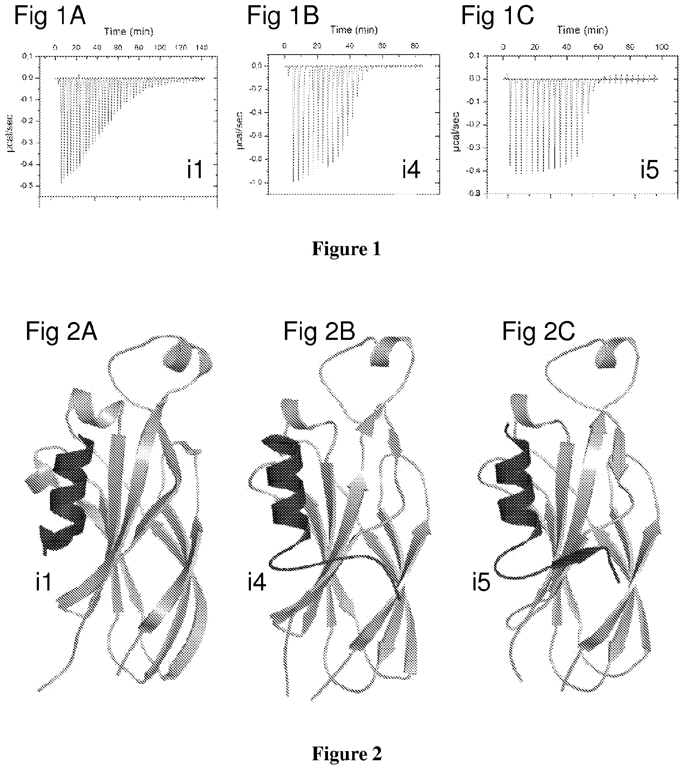

Ces différentes étapes de design ont permis d'augmenter l'affinité des peptides pour Asf1 d'un facteur 200 : partant d'un fragment de l'histone H3 seul (le peptide i1) montrant une affinité de 8µM, un inhibiteur i4 (SEQ ID No 42) a été obtenu, qui montre une affinité de 180nM ±40nM pour lequel les inventeurs ont résolu la structure du complexe avec Asf1 à haute résolution (Figure 2). Un autre peptide, i5 (SEQ ID No 32), a pu alors être conçu, présentant une affinité de 40nM ±13nM (Figure 1). A partir de ce peptide, un peptide contrôle a été conçu (i5 muté, SEQ ID No 68). Il a 90% d'identité avec le peptide i5, mais est muté sur trois résidus clés en alanine et ne se lie plus à Asf1. Ce peptide a été synthétisé pour servir de contrôle négatif dans les expériences de compétition ainsi que de test d'interaction. La séquence du peptide i5 a ensuite été optimisée de façon à obtenir les peptides i5_s3 (SEQ ID No 75), i5_D (SEQ ID No 76), i5_loopR (SEQ ID No 77), i5_loopA (SEQ ID No 78), i5_Aloop (SEQ ID No 79) et i6 (SEQ ID No 80) dont l'affinité est au moins aussi bonne que celle du peptide i5.

### Caractérisation biophysique et structurale:

Le design des peptides a été réalisé grâce à plusieurs allers-retours entre la caractérisation biophysique des propriétés de liaison du peptide et la modélisation moléculaire. L'impact des mutations sur la structuration a été systématiquement vérifié par dichroïsme circulaire et par RMN. Les structures tridimensionnelles des peptides i4 et i5 en interaction avec Asf1(1-156) ont été résolues par cristallographie des rayons X (Figure 2).

### Compétition peptide/histones in vitro:

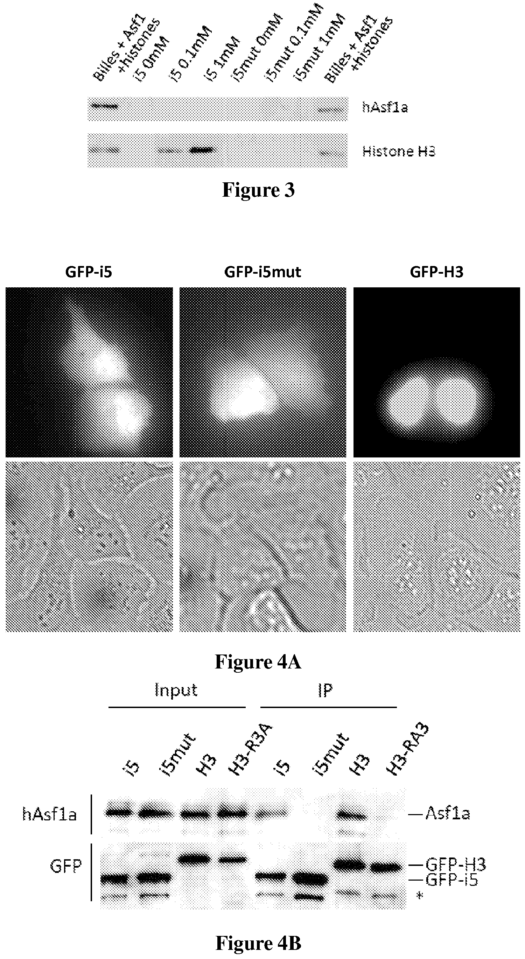

Les inventeurs ont contrôlé que le peptide inhibiteur est capable de rentrer en compétition avec les histones pour la liaison à Asf1 par des tests de compétition sur colonne GST. Pour cela, des billes de GST liées à Asf1 ont été incubées avec un excès d'histones H3-H4, puis lavées et incubées avec des concentrations croissantes de peptide. Les histones éluées ont été analysées par Western Blot. Lorsque les billes sont incubées avec seulement du tampon (0 mM i5 sur la Figure 3) ou avec le peptide i5muté (i5muté 0,1 et 1mM sur la Figure 3), les histones restent accrochées à Asf1. Par contre, lorsque les billes sont incubées avec 0,1 mM puis 1 mM de peptide i5, on a observé que des histones sont décrochées des billes (puits 0,1 mM i5 et 1 mM i5 sur la Figure 3). Ce résultat montre que, *in vitro,* le peptide est capable de dissocier l'interaction entre Asf1 et les histones.

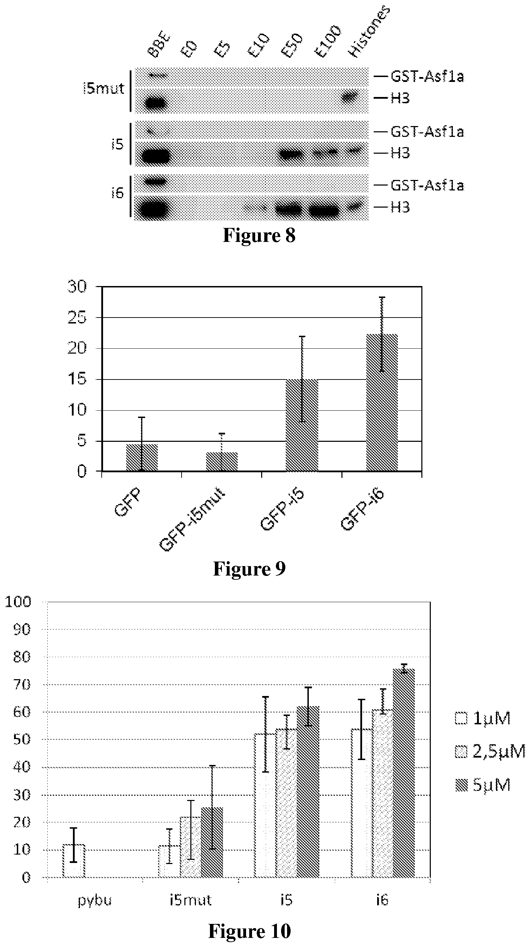

Lorsque la même expérience est réalisée avec différentes quantités de peptides i5 et i6 ainsi que le peptide contrôle i5mut (figure 8), on observe qu'une plus faible quantité du peptide i6 est suffisante pour dissocier le complexe Asf1-histones.

### Caractérisation de l'interaction Asf1-peptide in vivo:

Les inventeurs ont contrôlé que l'interaction entre Asf1 et les peptides peut s'établir dans des cellules humaines par co-immunoprécipitation (Figure 4). Pour cela, des cellules U2OS ont été transfectées grâce à de la Lipofectamine (Invitrogen) avec des plasmides contenant le gène des peptides i5 et i5mut, capables (ou non respectivement) de lier Asf1, sous forme d'une protéine de fusion avec la protéine fluorescente eGFP afin de contrôler la localisation du peptide par microscopie de fluorescence. La localisation des protéines de fusion a été analysée par microscopie de fluorescence (Figure 4A). Le contrôle positif est constitué par des cellules transfectées avec l'histone H3 entière également fusionnée à eGFP, tandis qu'un second contrôle négatif est constitué de cellules transfectées avec l'histone H3 mutée sur 3 résidus clés pour ne pas interagir avec Asf1 (H3-R3A). Dans ce système, on observe comme attendu une co-immunoprécipitation avec Asf1 (Figure 4B).

### Caractérisation du phénotype lié à l'ajout du peptide dans des cellules humaines:

Afin d'analyser l'effet biologique du peptide sur des cellules en culture, les inventeurs ont testé en même temps sa capacité à pénétrer dans les cellules. Le peptide a été vectorisé à l'aide d'une courte séquence d'environ 10 acides aminés en N terminal qui confère au peptide une propriété de « Cell Penetrating Peptide », c'est-à-dire la capacité d'être internalisé dans les cellules par un mécanisme dit de transduction. Cette séquence (RRP)₅ a été publiée par le groupe de Schepartz en 2007 (Journal Of The American Chemical Society 129(47): 14578).

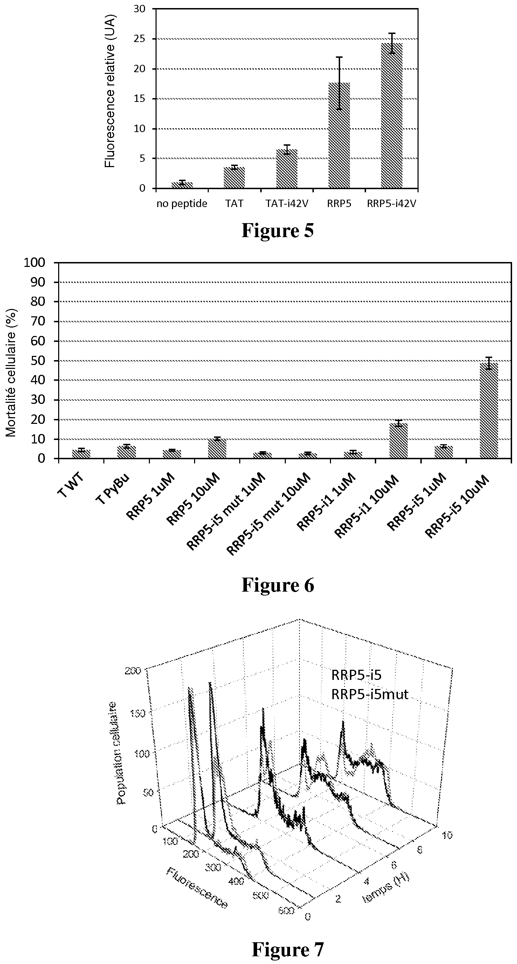

La capacité du peptide i42V (SEQ ID N° 47) couplée soit à la séquence TAT (peptide TAT-i42V, SEQ ID N° 58) ou à la séquence (RRP)₅ (peptide RRP5-i42V, SEQ ID N° 59) a été mesurée grâce à ces peptides couplés avec un marqueur FITC. La fluorescence moyenne des cellules a été analysée par cytométrie de flux (Figure 5). De façon intéressante, les peptides couplés aux séquences de pénétration pénètrent plus efficacement que les séquences de pénétration seules.

Afin d'optimiser l'internalisation du peptide, la transduction est effectuée en présence de pyrène butyrate, selon le protocole proposé par Takeuchi et al. (2006, ACS CHEMICAL BIOLOGY, 2006. 1(5): p. 299-303). Des peptides présentant des affinités diverses pour Asf1 ont été transduits, ainsi qu'un peptide contrôle ne liant pas Asf1 grâce à trois mutations sur des résidus clé pour l'interaction avec Asf1 (i5mut, SEQ ID N°68) dans les cellules U2OS. De façon remarquable, 24h après la transduction du peptide le plus affin à une concentration de 10 µM, une mortalité de 30% est observée alors qu'aucune toxicité n'est observée pour les cellules contrôle traitées soit avec du pyrène butyrate seul ou avec le peptide RRP5-i5mut qui le lie pas Asf1. De plus, cette toxicité est corrélée à l'affinité des peptides initialement mesurée par micro-calorimétrie, ce qui suggère fortement que la toxicité observée est bien reliée à l'inhibition de la protéine chaperon Asf1 (Figure 6). RRP5-i1 correspond au peptide de SEQ ID No 60. RRP5-i5 correspond au peptide de SEQ ID No 66. Cette corrélation entre l'affinité du peptide pour Asf1 et la toxicité cellulaire induite a été confirmée pour un autre type cellulaire (cellules Hela) et en utilisant un autre protocole pour mesurer la survie cellulaire, le test MTT (Figure 10).

Afin de vérifier que cette toxicité n'était pas un artéfact lié à la façon d'introduire le peptide dans la cellule, les inventeurs ont introduit le peptide sous forme d'un plasmide par transfection (nucleofection). Ils ont également observé une corrélation entre l'affinité du peptide pour Asf1 in vitro et la toxicité cellulaire (Figure 9).

La toxicité cellulaire pouvant résulter d'un ensemble de raisons très diverses, les inventeurs ont cherché à mettre en évidence un phénotype plus spécifiquement relié à l'inhibition du chaperon d'histones Asf1. Ils ont pour cela analysé l'effet de la transduction des peptides sur la progression du cycle cellulaire. Les cellules U2OS ont été synchronisées en phase G1. Après redémarrage du cycle et transduction des peptides RRP5-i5 (SEQ ID No 66) ou RRP5-i5mut (SEQ ID N°69) on constate que la transition entre les phases G1 et S est ralentie pour la version active du peptide et pas sa version muté (Figure 7), prouvant la spécificité d'action du peptide inhibiteur de l'interaction Asf1-Histone.

### Matériels et Méthodes

### Expression et clonage

Les protéines humaines Asf1a et b ainsi que le domaine N-terminal conservé (1-156) de chacune des isoformes ont été clonés dans le vecteur peTM30. Les protéines recombinantes, non marquées, marquées ¹⁵N et ¹³C/¹⁵N ont été purifiées d'après le protocole de Mousson et al (2004, Journal Of Biomolecular Nmr, 29(3): 413-414). Les peptides ont été soit produits par synthèse chimique (Genecust), soit clonés après une synthèse de gène (GENECUST) dans le vecteur Gateway pDest 17 (Invitrogen), puis purifiés d'après le protocole de Mousson et al (2005, Proc. Natl. Sci. USA, 102(17): p. 5975-5980).

### Conception

La conception des peptides a été effectuée en utilisant les logiciels Rosetta (Das et al, 2008, Annual Review Of Biochemistry, 77: p. 363-382), Fold-X (Guerois et al. 2002, J Mol Biol 320(2): 369-387) et AGADIR (Lacroix, et al 1998, J Mol Biol 284(1): 173-191).

### Dichroïsme circulaire :

Les expériences de dichroïsme circulaire ont été réalisées grâce à un appareil Jasco en utilisant une concentration des peptides à 50µM, dans H₂O à pH 5.5, à 5°C.

### Calorimétrie :

Toutes les expériences de calorimétrie ont été menées dans un tampon Tris 50mM pH7,4 à 5°C, sauf mention contraire. Les peptides ont été introduits dans la seringue à une concentration de 0,5mM ou 0,16mM, et Asf1 a été introduit dans la cuve à une concentration de 30 µM ou de 10µM. Des tests de calorimétrie ont aussi été menés à 25°C ou dans le tampon Tris 50mM NaCl 150mM pH7,4 pour obtenir des informations plus précises sur les caractéristiques des interactions étudiées.

### RMN :

Tous les échantillons de RMN ont été préparés soit dans un tampon H₂O, 10% D₂O, 0,1% NaN₃, 1mM EDTA 0.1% NaN₃, 0.1 mM 2,2-dimethyl-2-silapentane-5-sulfonate, pH5,5 pour les spectres RMN des peptides seuls, soit dans un tampon Tris D₁₁ 10mM, 0.1% NaN₃, 1 mM EDTA, 0.1 mM 2,2-dimethyl-2-silapentane-5-sulfonate, 10% D₂O, pH7,4. Plusieurs types d'échantillons ont été utilisés : Asf1a 1-156 uniformément marqué ¹⁵N ou ¹³C/¹⁵N, en complexe avec des peptides non marqués. Pour ces expériences, la concentration des échantillons était de 0.1mM. Les expériences de RMN ont été réalisées sur des spectromètres Bruker de 600 ou 700MHz équipés de cryosondes.

### Cristallographie :

Les cristaux de Asf1 en complexe avec le peptide inhibiteur i4 (SEQ ID No 60) ont été obtenus par diffusion de vapeur dans une solution de Tris/HCl 100mM pH7.5, 5% PEG 8000, 30% Glycérol.

Ceux du complexe avec le peptide inhibiteur i5 (SEQ ID No 66) ont été obtenus dans des conditions de LiSO₄ 250mM, 7% PEG 8000, Acide Citrique 100mM pH2.5, Xylitol 22.5%.

La structure des complexes a été résolue par remplacement moléculaire à l'aide de la structure cristallographique de Asf1a (code Pdb 2io5)

### Tests de compétition sur colonne GST :

La protéine humaine GST-Asf1 1-156 a été produite puis incubée avec des billes GST (glutathione S Transferase, Sigma). Pour chaque expérience, 40 µg d'Asf1 recombinant fixés sur des billes d'agarose ont été incubés avec 400ug d'histones. Après lavage avec le tampon Tris 50mM pH8, NaCl 150mM, EDTA 1mM, DTT 1mM, NP40 0.5%, les billes ont été incubées avec différentes concentrations de peptide (0 à 1mM) solubilisé dans le même tampon. La quantité d'histones dissociée a été révélée par Western Blot et des anticorps anti-GST et anti-H3 (AbCam).

### Co-Immuno Précipitations :

Des cellules U2OS ont été transfectées avec des plasmides contenant des histones ou le peptide, taggé à la EYFP. 24h après les cellules ont été lysées puis incubées sur des billes comprenant un anticorps anti-GFP (Miltenyi Biotec). Après lavage dans un tampon Acétate de potassium 100mM, 30mM KCl, 10mM NaH₂PO₄, 1mM MgCl₂, Triton X100 0.2%, NaCl 200mM, les protéines associées aux billes ont été analysées par Western Blot, avec des anticorps anti-GFP (AbCam) et anti-Asf1 (produit par l'équipe de Carl Mann).

### Test de pénétration des cellules

Des cellules U2OS ont été incubées avec du milieu de culture complété avec du peptide fluorescent à une concentration finale de 1µM pendant une heure puis lavées avec du tampon PBS et détachées par l'ajout de trypsine, lavées avec un tampon PBS puis analysées par cytométrie de flux (FACS)

### Tests de toxicité et phénotypes:

Pour tester les phénotypes induits par le peptide, un protocole récemment développé pour le « Cell Penetrating TAT » a été utilisé. Les peptides sont solubilisés dans un tampon PBS contenant 50µM de pyrène butyrate et incubés avec les cellules U2OS (ou Hela) pendant 4 minutes. Les cellules sont ensuite lavées 3 fois avec du tampon PBS puis reprises dans du milieu de culture contenant du serum de veau fetal. La mortalité cellulaire a été analysée par FACS 24 à 48h plus tard par l'incorporation d'iodure de propidium ou grâce à un test MTT (bromure de 3-(4,5-dimethylthiazol-2-yl)-2,5-diphenyl tetrazolium). (Mosmann, T. J. Immunol. Methods 65: 55-63, 1983).

### Nucleofection

Les cellules U2OS ont été transfectée avec un vecteur portant le gène des peptides inhibiteurs sous forme de protéine de fusion avec la GFP en utilisant un protocole de nucleofection (Nucleofector™ Solution V, Lonza) selon le protocole donné par le fournisseur.

### Test de perturbation du cycle

Des cellules U2OS ont été synchronisées en phase G1 par l'incubation pendant une nuit avec de la mimosine (200µM). Le redémarrage du cycle est déclenché par un lavage PBS. Les peptides solubilisés dans un tampon PBS contenant 50µM de pyrène butyrate ont ensuite été incubés avec les cellules pendant 4 minutes, les cellules lavées 3 fois avec du tampon PBS puis reprises dans du milieu de culture contenant du serum de veau fetal. Elles sont récoltées 1H, 4H, 7H et 10H plus tard. Le milieu est éliminé et le culot cellulaire repris dans 500µL de PBS + IP (iodure de propiduim) 40µg/mL + RNAse 40µg/mL. Après une incubation de 20 minutes à 4°C, la fluorescence des cellules est analysée par cytométrie de flux (FACS).

**Tableau de listage de séquences**

| SEQ ID No | Description |
|---|---|
| 1 | E1 de formule (I), mode a1) |
| 2 | E1 de formule (I), mode a2) |
| 3 | B de formule (II) |
| 4 | E2 de formule (III) |
| 5 | E1-B-E2 de formule (IV) |
| 6 | E1-B-E2 de formule (V) |
| 7 | E1 de formule (I), mode a1) |
| 8 | E1 de formule (I), mode a2) |
| 9 | E1-B-E2 de formule (VI) |
| 10 | E1-B-E2 de formule (VII) |
| 11 | E1-B-E2 de formule (VIII) |
| 12 | E1-B-E2 de formule (IX) |
| 13 | élément E1-B-E2 |
| 14 | élément E1-B-E2 |
| 15 | élément E1-B-E2 |
| 16 | élément E1-B-E2 |
| 17 | élément E1-B-E2 |
| 18 | élément E1-B-E2 |
| 19 | élément E1-B-E2 |
| 20 | élément E1-B-E2 |
| 21 | élément E1-B-E2 |
| 22 | élément E1-B-E2 |
| 23 | élément E1-B-E2 |
| 24 | élément E1-B-E2 |
| 25 | élément E1-B-E2 |
| 26 | élément E1-B-E2 |
| 27 | élément E1-B-E2 |
| 28 | élément E1-B-E2 |
| 29 | élément E1-B-E2 |
| 30 | élément E1-B-E2 |
| 31 | élément E1-B-E2 |
| 32 | élément E1-B-E2 - peptide i5 |
| 33 | élément E1-B-E2 |
| 34 | peptide de Tat |
| 35 | penetratin |
| 36 | poly-arginine |
| 37 | (RRP)₅ |
| 38 | peptide inhibiteur Asf1 / H3-H4-peptide i4_1 |
| 39 | peptide inhibiteur Asf1 / H3-H4 |
| 40 | peptide inhibiteur Asf1 / H3-H4-peptide i4_2 |
| 41 | peptide inhibiteur Asf1 / H3-H4 |
| 42 | peptide inhibiteur Asf1 / H3-H4- peptide i4 |
| 43 | peptide inhibiteur Asf1 / H3-H4 |
| 44 | peptide inhibiteur Asf1 / H3-H4 |
| 45 | peptide inhibiteur Asf1 / H3-H4 |
| 46 | peptide inhibiteur Asf1 / H3-H4 |
| 47 | peptide inhibiteur Asf1 / H3-H4-peptide i42V |
| 48 | peptide inhibiteur Asf1 / H3-H4-peptide i4V |
| 49 | peptide inhibiteur Asf1 / H3-H4 |
| 50 | peptide inhibiteur Asf1 / H3-H4-peptide RRP5-i42V-CA |
| 51 | peptide inhibiteur Asf1 / H3-H4 |
| 52 | peptide inhibiteur Asf1 / H3-H4 |
| 53 | peptide inhibiteur Asf1 / H3-H4 |
| 54 | peptide inhibiteur Asf1 / H3-H4 |
| 55 | peptide inhibiteur Asf1 / H3-H4 |
| 56 | peptide inhibiteur Asf1 / H3-H4 |
| 57 | peptide inhibiteur Asf1 / H3-H4 |
| 58 | peptide inhibiteur Asf1 / H3-H4-peptide TAT-i42V |
| 59 | peptide inhibiteur Asf1 / H3-H4-peptide RRP5-i42V |
| 60 | peptide inhibiteur Asf1 / H3-H4-peptide RRP5-i1 |
| 61 | peptide inhibiteur Asf1 / H3-H4 |
| 62 | peptide inhibiteur Asf1 / H3-H4 |
| 63 | peptide inhibiteur Asf1 / H3-H4 |
| 64 | peptide inhibiteur Asf1 / H3-H4 |
| 65 | peptide inhibiteur Asf1 / H3-H4 |
| 66 | peptide inhibiteur Asf1 / H3-H4-peptide RRP5-i5 |
| 67 | peptide inhibiteur Asf1 / H3-H4 |
| 68 | Peptide i5 muté |
| 69 | Peptide RRP5-i5 muté |
| 70 | Peptide i1 |
| 71 | Fragment H3 stabilisé |
| 72 | Fragment H3 stabilisé |
| 73 | peptide inhibiteur Asf1 / H3-H4 |
| 74 | peptide inhibiteur Asf1 / H3-H4 |
| 75 | Peptide i5_s3 |
| 76 | Peptide i5_D |
| 77 | Peptide i5_loopR |
| 78 | Peptide i5_loopA |
| 79 | Peptide i5_Aloop |
| 80 | Peptide i6 |
| 81 | Peptide RRP5-i6 |
| 82 | E1-B-E2 de formule (X) |

## Revendications

1. Molécule peptidique capable d'inhiber la formation du complexe entre Asf1 (Anti-Silencing Factor 1) et les histones H3-H4 et comprenant les éléments E1-B-E2, dans laquelle
a) E1 est un peptide de formule (I)
X₁-X₂-X₃-X₄-X₅-K-X₆-X₇-X₈- L-X₉-X₁₀-R-I-X₁₁ (I)
dans laquelle
a1) soit (SEQ ID No 1)
X₁ et X₂ sont absents ;
X₃ est I, R ou L, de préférence I ou L ;
X₄ est T ou M, de préférence T ;
X₅ est P ;
X₆ est E ou D, de préférence D ;
X₇ est sélectionné parmi le groupe consistant en R, A, I et E, de préférence R, A et E ;
X₈ est sélectionné parmi le groupe consistant en R, Q et E ;
X₉ est R ou A ;
X₁₀ est R ; et
X₁₁ est R ou E ;
a2) soit (SEQ ID No 2)
X₁, X₇ et X₉ sont A ;
X₂ est S ;
X₃ est T ;
X₄, X₅, et X₈ sont E ou R ;
X₆ est W ; et
X₁₀ est R ;
et X₁₁ sont R ou A ;
b) B est un peptide de formule (II)
G-X₁₂-G-X₁₃-X₁₄ (II)
(SEQ ID No 3) dans laquelle
X₁₂ est A ou P ;
X₁₃ est absent ou sélectionné parmi les acides aminés G, A et S ;
X₁₄ est absent ou S ; et
c) E2 est un peptide de formule (III)
X₁₅-X₁₆-X₁₇-X₁₈-G-X₁₉-X₂₀ (III) (SEQ ID No 4)
dans laquelle
X₁₅ est R, V ou A ;
X₁₆ est T ou V ;
X₁₇ est L ;
X₁₈ est Y, N ou D ;
X₁₉ est sélectionné par le groupe consistant en F ou M ; et
X₂₀ est sélectionné par le groupe consistant en G, Q et N.

2. Molécule peptidique capable d'inhiber la formation du complexe entre Asf1 (Anti-Silencing Factor 1) et les histones H3-H4 et comprenant les éléments E1-B-E2, dans laquelle les éléments E1-B-E2 présentent l'une des séquences suivantes
X₃-X₄-P-K-X₆-X₇-X₈-L-X₉-R-R-I-X₁₁-G-X₁₂-G-X₁₃-X₁₄-X₁₅-X₁₆-L-X₁₈-G-X₁₉-X₂₀ (VIII) (SEQ ID No 11)
dans laquelle
X₃ est I, R ou L, de préférence I ou L ;
X₄ est T ou M, de préférence T ;
X₆ est E ou D, de préférence D ;
X₇ est sélectionné parmi le groupe consistant en R, A, I et E, de préférence parmi R, A etE;
X₈ est sélectionné parmi le groupe consistant en R, Q et E ;
X₉ est R ou A ;
X₁₁ est R ou E ;
X₁₂ est A ou P ;
X₁₃ est absent ou sélectionné parmi les acides aminés G, A et S ;
X₁₄ est absent ou S ;
X₁₅ est R ou V ;
X₁₆ est T ou V ;
X₁₈ est Y, N ou D ;
X₁₉ est F, M, A ou Q, de préférence F ou M, de manière encore plus préféré F ; et
X₂₀ est sélectionné par le groupe consistant en G, Q et N ;
A-S-T-X₄-R-K-W-A-E-L-A-R-R-I-R-G-X₁₂-G-X₁₃-X₁₄-X₁₅-X₁₆-L-X₁₈-G-X₁₉-X₂₀ (IX) (SEQ ID No 12)
dans laquelle
X₄ est E ou R ;
X₁₂ est A ou P ;
X₁₃ est absent ou sélectionné parmi les acides aminés G, A et S ;
X₁₄ est absent ou S ;
X₁₅ est R ou V ;
X₁₆ est T ou V ;
X₁₈ est Y, N ou D ;
X₁₉ est F, M, A ou Q, de préférence F ou M, de manière encore plus préféré F ; et
X₂₀ est sélectionné par le groupe consistant en G, Q et N,
A-S-T-X₄-X₅-K-W-A-E-L-A-X₈-R-I-X₁₁-G-X₁₂-G-X₁₃-X₁₄-X₁₅-X₁₆-L-X₁₈-G-X₁₉-X₂₀ (XI) (SEQ ID No 82)
dans laquelle
X₄, X₅, X₈ sont E ou R ;
X₁₁ est R ou A ;
X₁₂ est A ou P ;
X₁₃ est absent ou sélectionné parmi les acides aminés G, A et S ;
X₁₄ est absent ou S ;
X₁₅ est R, V ou A ;
X₁₆ est T ou V ;
X₁₈ est Y, N ou D ;
X₁₉ est F ou M, de préférence F ; et
X₂₀ est sélectionné par le groupe consistant en G, Q et N.

3. La molécule selon l'une quelconque des revendications 1-2, dans laquelle les éléments E1-B-E2 présentent l'une des séquences suivantes
IMPKDIQLARRIRGAGGRTLYGFG (SEQ ID No 13)
IMPKDIQLARRIRGAGASRTLYGFG (SEQ ID No 14)
ITPKEAQLARRIRGAGGRTLNGFG (SEQ ID No 15)
ITPKEAQLARRIRGAGASRTLNGFG (SEQ ID No 16)
LTPKEAELARRIRGAGGRTLNGFG (SEQ ID No 17)
LTPKEAELARRIRGAGASRTLNGFG (SEQ ID No 18)
ITPKEAQLARRIEGAGASVTLNGFG (SEQ ID No 19)
LTPKEAELARRIEGAGASVTLNGFG (SEQ ID No 20)
ITPKEEQLRRRIEGAGASVTLNGFG (SEQ ID No 21)
ITPKEAQLARRIRGAGGVTLNGFG (SEQ ID No 22)
LTPKEAELARRIRGAGGVTLNGFG (SEQ ID No 23)
LTPKEAELARRIRGAGRTLNGFG (SEQ ID No 24)
LTPKEAELARRIRGAGGRTLNGFG (SEQ ID No 25)
LTPKEAELARRIRGAGASRTLNGFG (SEQ ID No 26)
RTPKERRLARRIRGAGGRTLNGFG (SEQ ID No 27)
RTPKEARLARRIRGAGGRTLNGFG (SEQ ID No 28)
LTPKEAELARRIRGAGGVTYDGFG (SEQ ID No 29)
LTPKEAELARRIRGAGGVTLNGAN (SEQ ID No 30)
STERKWAELARRIRGAGGVTLNGFG (SEQ ID No 31)
ASTERKWAELARRIRGAGGVTLNGFG (SEQ ID No 32)
ASTRRKWAELARRIRGAGGVTLNGFG (SEQ ID No 33)
ASTEEKWARLARRIRGAGGVTLNGFG (SEQ ID No 75)
ASTERKWAELARRIRGAGGVTLDGFG (SEQ ID No 76)
ASTERKWAELARRIRGAGGRTLNGFG (SEQ ID No 77)
ASTERKWAELARRIRGAGGATLNGFG (SEQ ID No 78)
ASTERKWAELARRIAGAGGVTLNGFG (SEQ ID No 79)
ASTEEKWARLARRIAGAGGVTLDGFG (SEQ ID No 80)
ainsi qu'une séquence présentant 90 ou 95 % d'identité avec celles-ci.

4. La molécule selon l'une quelconque des revendications 1-3, dans laquelle la molécule comprend en outre un peptide facilitant la pénétration cellulaire.

5. La molécule selon la revendication 4, dans laquelle le peptide facilitant la pénétration cellulaire est RRPRRPRRPRRPRRP (SEQ ID No 37).

6. La molécule selon l'une quelconque des revendications 1-5, la molécule comprenant ou consistant en une séquence sélectionnée parmi le groupe consistant en
GAMGTIMPKDIQLARRIRGAGGRTLYGFG (SEQ ID No 38)
GAMGTIMPKDIQLARRIRGAGASRTLYGFG (SEQ ID No 39)
GAMGTITPKEAQLARRIRGAGGRTLNGFG (SEQ ID No 40)
GAMGTITPKEAQLARRIRGAGASRTLNGFG (SEQ ID No 41)
GAMGTLTPKEAELARRIRGAGGRTLNGFG (SEQ ID No 42)
GAMGTLTPKEAELARRIRGAGASRTLNGFG (SEQ ID No 43)
GAMGTITPKEAQLARRIEGAGASVTLNGFG (SEQ ID No 44)
GAMGTLTPKEAELARRIEGAGASVTLNGFG (SEQ ID No 45)
GAMGTITPKEEQLRRRIEGAGASVTLNGFG (SEQ ID No 46)
GAMGTITPKEAQLARRIRGAGGVTLNGFG (SEQ ID No 47)
GAMGTLTPKEAELARRIRGAGGVTLNGFG (SEQ ID No 48)
GAMGTLTPKEAELARRIRGAGRTLNGFG (SEQ ID No 49)
GAMGTLTPKEAELARRIRGAGASRTLNGFG (SEQ ID No 51)
GAMGLTAAEHAKRSTLTPKEAQLARRIRGAGGVTLNGFG (SEQ ID No 52)
LTAAEHAKRSTLTPKEAELARRIRGAGGVTLNGFG (SEQ ID No 53)
LTAAEHAKRSTLTPKEAQLARRIEGAGASVTLNGFG (SEQ ID No 54)
LTAAEHAKRSTLTPKEAELARRIEGAGASVTLNGFG (SEQ ID No 55)
GAMGTRTPKERRLARRIRGAGGRTLNGFG (SEQ ID No 56)
GAMGTRTPKEARLARRIRGAGGRTLNGFG (SEQ ID No 57)
GRKKRRQRRRGAMGTITPKEAQLARRIRGAGGVTLNGFG (SEQ ID No 58)
RRPRRPRRPRRPRRPGAMGTITPKEAQLARRIRGAGGVTLNGFG (SEQ ID No 59)
GAMGTLTPKEAELARRIRGAGGVTYDGFG (SEQ ID No 61)
GAMGTLTPKEAELARRIRGAGGVTLNGFGASTG (SEQ ID No 62)
GAMGTLTPKEAELARRIRGAGGVTLNGANFVSTG (SEQ ID No 63)
GAMGRVPPAVRKLGNSTERKWAELARRIRGAGGVTLNGFG (SEQ ID No 64)
GAMGRVPPAVRKLGNASTERKWAELARRIRGAGGVTLNGFG (SEQ ID No 65)
ASTERKWAELARRIRGAGGVTLNGFG (SEQ ID No 32)
ASTRRKWAELARRIRGAGGVTLNGFG (SEQ ID No 33)
ASTEEKWARLARRIRGAGGVTLNGFG (SEQ ID No 75)
ASTERKWAELARRIRGAGGVTLDGFG (SEQ ID No 76)
ASTERKWAELARRIRGAGGRTLNGFG (SEQ ID No 77)
ASTERKWAELARRIRGAGGATLNGFG (SEQ ID No 78)
ASTERKWAELARRIAGAGGVTLNGFG (SEQ ID No 79)
ASTEEKWARLARRIAGAGGVTLDGFG (SEQ ID No 80)
RRPRRPRRPRRPRRPASTERKWAELARRIRGAGGVTLNGFGGCA (SEQ ID No 66)
RRPRRPRRPRRPRRPASTERKWAELARRIRGAGGVTLNGFG (SEQ ID No 67),
RRPRRPRRPRRPRRPGAMGTITPKEAQLARRIRGAGGVTLNGFGCA (SEQ ID No 50) et
RRPRRPRRPRRPRRPASTEEKWARLARRIAGAGGVTLDGFG (SEQ ID No 81)
ainsi qu'une séquence présentant 90 ou 95 % d'identité avec celles-ci.

7. La molécule selon l'une quelconque des revendications 1-6, la molécule comprenant ou consistant en une séquence sélectionnée parmi le groupe consistant en
RRPRRPRRPRRPRRPGAMGTITPKEAQLARRIRGAGGVTLNGFG (SEQ ID No 59)
RRPRRPRRPRRPRRPASTERKWAELARRIRGAGGVTLNGFGGCA (SEQ ID No 66)
RRPRRPRRPRRPRRPASTERKWAELARRIRGAGGVTLNGFG (SEQ ID No 67)
RRPRRPRRPRRPRRPGAMGTITPKEAQLARRIRGAGGVTLNGFGCA (SEQ ID No 50) et
RRPRRPRRPRRPRRPASTEEKWARLARRIAGAGGVTLDGFG (SEQ ID No 81).

8. La molécule selon l'une quelconque des revendications 1-7 en tant que médicament.

9. Composition pharmaceutique comprenant une molécule selon l'une quelconque des revendications 1-7.

10. La composition pharmaceutique selon la revendication 9, dans laquelle la composition comprend un autre composé d'intérêt thérapeutique.

11. La molécule selon l'une quelconque des revendications 1-7 pour une utilisation dans le traitement d'un cancer.

12. La molécule pour une utilisation selon la revendication 11, la molécule étant utilisée en combinaison avec un autre composé d'intérêt thérapeutique ou avec une radiothérapie.

## Patentansprüche

1. Peptidmolekül, das in der Lage ist, die Bildung des Komplexes zwischen Asfl (Anti-Silencing Factor 1) und den Histonen H3-H4 zu hemmen, und die Elemente E1-B-E2 umfasst, wobei
a) E1 ein Peptid der Formel (I)
X₁-X₂-X₃-X₄-X₅-K-X₆-X₇-X₈- L-X₉-X₁₀-R-I-X₁₁ (I)
ist, wobei
a1) entweder (SEQ ID NO: 1)
X₁ und X₂ fehlen;
X₃ I, R oder L, bevorzugt I oder L, ist;
X₄ T oder M, bevorzugt T, ist;
X₅ P ist;
X₆ E oder D, bevorzugt D, ist;
X₇ aus der Gruppe ausgewählt ist, bestehend aus R, A, I und E, bevorzugt R, A und E;
X₈ aus der Gruppe ausgewählt ist, bestehend aus R, Q und E;
X₉ R oder A ist;
X₁₀ R ist; und
X₁₁ R oder E ist;
a2) oder (SEQ ID NO: 2)
X₁, X₇ und X₉ A sind;
X₂ S ist;
X₃ T ist;
X₄, X₅, und X₈ E oder R sind;
X₆ W ist; und
X₁₀ R ist;
und X₁₁ R oder A ist;
b) B ein Peptid der Formel (II)
G-X₁₂-G-X₁₃-X₁₄ (II) (SEQ ID NO: 3)
ist, wobei
X₁₂ A oder P ist;
X₁₃ fehlt oder aus den Aminosäuren G, A und S ausgewählt ist;
X₁₄ fehlt oder S ist;
und
c) E2 ein Peptid der Formel (III)
X₁₅-X₁₆-X₁₇-X₁₈-G-X₁₉-X₂₀ (III) (SEQ ID NO: 4)
ist, wobei
X₁₅ R, V oder A ist;
X₁₆ T oder V ist;
X₁₇ L ist;
X₁₈ Y, N oder D ist;
X₁₉ aus der Gruppe ausgewählt ist, bestehend aus F oder M; und
X₂₀ aus der Gruppe ausgewählt ist, bestehend aus G, Q und N.

2. Peptidmolekül, das in der Lage ist, die Bildung des Komplexes zwischen Asfl (Anti-Silencing Factor 1) und den Histonen H3-H4 zu hemmen, und die Elemente E1-B-E2 umfasst, wobei die Elemente E1-B-E2 eine der folgenden Sequenzen aufweisen
X₃-X₄-P-K-X₆-X₇-X₈-L-X₉-R-R-I-X₁₁-G-X₁₂-G-X₁₃-X₁₄-X₁₅-X₁₆-L-X₁₈-G-X₁₉-X₂₀ (VIII) (SEQ ID NO: 11)
wobei
X₃ I, R oder L, bevorzugt I oder L, ist;
X₄ T oder M, bevorzugt T, ist;
X₆ E oder D, bevorzugt D, ist;
X₇ aus der Gruppe ausgewählt ist, bestehend aus R, A, I und E, bevorzugt aus R, A und E ausgewählt ist;
X₈ aus der Gruppe ausgewählt ist, bestehend aus R, Q und E;
X₉ R oder A ist;
X₁₁ R oder E ist;
X₁₂ A oder P ist;
X₁₃ fehlt oder aus den Aminosäuren G, A und S ausgewählt ist;
X₁₄ fehlt oder S ist;
X₁₅ R oder V ist;
X₁₆ T oder V ist;
X₁₈ Y, N oder D ist;
X₁₉ F, M, A oder Q, bevorzugt F oder M, noch bevorzugter F, ist; und
X₂₀ aus der Gruppe ausgewählt ist, bestehend aus G, Q und N;
A-S-T-X₄-R-K-W-A-E-L-A-R-R-I-R-G-X₁₂-G-X₁₃-X₁₄-X₁₅-X₁₆-L-X₁₈-G-X₁₉-X₂₀ (IX) (SEQ ID NO: 12)
wobei
X₄ E oder R ist;
X₁₂ A oder P ist;
X₁₃ fehlt oder aus den Aminosäuren G, A und S ausgewählt ist;
X₁₄ fehlt oder S ist;
X₁₅ R oder V ist;
X₁₆ T oder V ist;
X₁₈ Y, N oder D ist;
X₁₉ F, M, A oder Q, bevorzugt F oder M, noch bevorzugter F, ist; und
X₂₀ aus der Gruppe ausgewählt ist, bestehend aus G, Q und N,
A-S-T-X₄-X₅-K-W-A-E-L-A-X₈-R-I-X₁₁-G-X₁₂-G-X₁₃-X₁₄-X₁₅-X₁₆-L-X₁₈-G-X₁₉-X₂₀ (XI) (SEQ ID NO: 82)
wobei
X₄, X₅, X₈ E oder R sind;
X₁₁ R oder A ist;
X₁₂ A oder P ist;
X₁₃ fehlt oder aus den Aminosäuren G, A und S ausgewählt ist;
X₁₄ fehlt oder S ist;
X₁₅ R, V oder A ist;
X₁₆ T oder V ist;
X₁₈ Y, N oder D ist;
X₁₉ F oder M, bevorzugt F, ist; und
X₂₀ aus der Gruppe ausgewählt ist, bestehend aus G, Q und N.

3. Das Molekül gemäß einem der Ansprüche 1 bis 2, wobei die Elemente E1-B-E2 eine der folgenden Sequenzen aufweisen:
IMPKDIQLARRIRGAGGRTLYGFG (SEQ ID NO: 13)
IMPKDIQLARRIRGAGASRTLYGFG (SEQ ID NO: 14)
ITPKEAQLARRIRGAGGRTLNGFG (SEQ ID NO: 15)
ITPKEAQLARRIRGAGASRTLNGFG (SEQ ID NO: 16)
LTPKEAELARRIRGAGGRTLNGFG (SEQ ID NO: 17)
LTPKEAELARRIRGAGASRTLNGFG (SEQ ID NO: 18)
ITPKEAQLARRIEGAGASVTLNGFG (SEQ ID NO: 19)
LTPKEAELARRIEGAGASVTLNGFG (SEQ ID NO: 20)
ITPKEEQLRRRIEGAGASVTLNGFG (SEQ ID NO: 21)
ITPKEAQLARRIRGAGGVTLNGFG (SEQ ID NO: 22)
LTPKEAELARRIRGAGGVTLNGFG (SEQ ID NO: 23)
LTPKEAELARRIRGAGRTLNGFG (SEQ ID NO: 24)
LTPKEAELARRIRGAGGRTLNGFG (SEQ ID NO: 25)
LTPKEAELARRIRGAGASRTLNGFG (SEQ ID NO: 26)
RTPKERRLARRIRGAGGRTLNGFG (SEQ ID NO: 27)
RTPKEARLARRIRGAGGRTLNGFG (SEQ ID NO: 28)
LTPKEAELARRIRGAGGVTYDGFG (SEQ ID NO: 29)
LTPKEAELARRIRGAGGVTLNGAN (SEQ ID NO: 30)
STERKWAELARRIRGAGGVTLNGFG (SEQ ID NO: 31)
ASTERKWAELARRIRGAGGVTLNGFG (SEQ ID NO: 32)
ASTRRKWAELARRIRGAGGVTLNGFG (SEQ ID NO: 33)
ASTEEKWARLARRIRGAGGVTLNGFG (SEQ ID NO: 75)
ASTERKWAELARRIRGAGGVTLDGFG (SEQ ID NO: 76)
ASTERKWAELARRIRGAGGRTLNGFG (SEQ ID NO: 77)
ASTERKWAELARRIRGAGGATLNGFG (SEQ ID NO: 78)
ASTERKWAELARRIAGAGGVTLNGFG (SEQ ID NO: 79)
ASTEEKWARLARRIAGAGGVTLDGFG (SEQ ID NO: 80)
sowie eine Sequenz, die zu 90 oder 95% mit besagten Sequenzen identisch ist.

4. Das Molekül gemäß einem der Ansprüche 1 bis 3, wobei das Molekül außerdem ein Peptid umfasst, das die Zellpenetration erleichtert.

5. Das Molekül gemäß Anspruch 4, wobei das Peptid, das die Zellpenetration erleichtert, RRPRRPRRPRRPRRP (SEQ ID NO: 37) ist.

6. Das Molekül gemäß einem der Ansprüche 1 bis 5, wobei das Molekül aus einer Sequenz besteht oder eine Sequenz umfasst, ausgewählt aus der Gruppe, bestehend aus
GAMGTIMPKDIQLARRIRGAGGRTLYGFG (SEQ ID NO: 38)
GAMGTIMPKDIQLARRIRGAGASRTLYGFG (SEQ ID NO: 39)
GAMGTITPKEAQLARRIRGAGGRTLNGFG (SEQ ID NO: 40)
GAMGTITPKEAQLARRIRGAGASRTLNGFG (SEQ ID NO: 41)
GAMGTLTPKEAELARRIRGAGGRTLNGFG (SEQ ID NO: 42)
GAMGTLTPKEAELARRIRGAGASRTLNGFG (SEQ ID NO: 43)
GAMGTITPKEAQLARRIEGAGASVTLNGFG (SEQ ID NO: 44)
GAMGTLTPKEAELARRIEGAGASVTLNGFG (SEQ ID NO: 45)
GAMGTITPKEEQLRRRIEGAGASVTLNGFG (SEQ ID NO: 46)
GAMGTITPKEAQLARRIRGAGGVTLNGFG (SEQ ID NO: 47)
GAMGTLTPKEAELARRIRGAGGVTLNGFG (SEQ ID NO: 48)
GAMGTLTPKEAELARRIRGAGRTLNGFG (SEQ ID NO: 49)
GAMGTLTPKEAELARRIRGAGASRTLNGFG (SEQ ID NO: 51)
GAMGLTAAEHAKRSTLTPKEAQLARRIRGAGGVTLNGFG (SEQ ID NO: 52)
LTAAEHAKRSTLTPKEAELARRIRGAGGVTLNGFG (SEQ ID NO: 53)
LTAAEHAKRSTLTPKEAQLARRIEGAGASVTLNGFG (SEQ ID NO: 54)
LTAAEHAKRSTLTPKEAELARRIEGAGASVTLNGFG (SEQ ID NO: 55)
GAMGTRTPKERRLARRIRGAGGRTLNGFG (SEQ ID NO: 56)
GAMGTRTPKEARLARRIRGAGGRTLNGFG (SEQ ID NO: 57)
GRKKRRQRRRGAMGTITPKEAQLARRIRGAGGVTLNGFG (SEQ ID NO: 58)
RRPRRPRRPRRPRRPGAMGTITPKEAQLARRIRGAGGVTLNGFG (SEQ ID NO: 59)
GAMGTLTPKEAELARRIRGAGGVTYDGFG (SEQ ID NO: 61)
GAMGTLTPKEAELARRIRGAGGVTLNGFGASTG (SEQ ID NO: 62)
GAMGTLTPKEAELARRIRGAGGVTLNGANFVSTG (SEQ ID NO: 63)
GAMGRVPPAVRKLGNSTERKWAELARRIRGAGGVTLNGFG (SEQ ID NO: 64)
GAMGRVPPAVRKLGNASTERKWAELARRIRGAGGVTLNGFG (SEQ ID NO: 65)
ASTERKWAELARRIRGAGGVTLNGFG (SEQ ID NO: 32)
ASTRRKWAELARRIRGAGGVTLNGFG (SEQ ID NO: 33)
ASTEEKWARLARRIRGAGGVTLNGFG (SEQ ID NO: 75)
ASTERKWAELARRIRGAGGVTLDGFG (SEQ ID NO: 76)
ASTERKWAELARRIRGAGGRTLNGFG (SEQ ID NO: 77)
ASTERKWAELARRIRGAGGATLNGFG (SEQ ID NO: 78)
ASTERKWAELARRIAGAGGVTLNGFG (SEQ ID NO: 79)
ASTEEKWARLARRIAGAGGVTLDGFG (SEQ ID NO: 80) RRPRRPRRPRRPRRPASTERKWAELARRIRGAGGVTLNGFGGCA (SEQ ID NO: 66)
RRPRRPRRPRRPRRPASTERKWAELARRIRGAGGVTLNGFG (SEQ ID NO: 67),
RRPRRPRRPRRPRRPGAMGTITPKEAQLARRIRGAGGVTLNGFGCA (SEQ ID NO: 50)
und
RRPRRPRRPRRPRRPASTEEKWARLARRIAGAGGVTLDGFG (SEQ ID NO: 81)
sowie eine Sequenz, die zu 90 oder 95% mit besagten Sequenzen identisch ist.

7. Das Molekül gemäß einem der Ansprüche 1 bis 6, wobei das Molekül aus einer Sequenz besteht oder eine Sequenz umfasst, ausgewählt aus der Gruppe, bestehend aus
RRPRRPRRPRRPRRPGAMGTITPKEAQLARRIRGAGGVTLNGFG (SEQ ID NO: 59)
RRPRRPRRPRRPRRPASTERKWAELARRIRGAGGVTLNGFGGCA (SEQ ID NO: 66)
RRPRRPRRPRRPRRPASTERKWAELARRIRGAGGVTLNGFG (SEQ ID NO: 67)
RRPRRPRRPRRPRRPGAMGTITPKEAQLARRIRGAGGVTLNGFGCA (SEQ ID NO: 50)
und
RRPRRPRRPRRPRRPASTEEKWARLARRIAGAGGVTLDGFG (SEQ ID NO: 81).

8. Das Molekül gemäß einem der Ansprüche 1 bis 7 als Medikament.

9. Pharmazeutische Zusammensetzung, umfassend ein Molekül gemäß einem der Ansprüche 1 bis 7.

10. Die pharmazeutische Zusammensetzung gemäß Anspruch 9, wobei die Zusammensetzung außerdem eine weitere Verbindung von therapeutischem Interesse umfasst.

11. Das Molekül gemäß einem der Ansprüche 1 bis 7 zur Verwendung in der Behandlung von Krebs.

12. Das Molekül zur Verwendung gemäß Anspruch 11, wobei das Molekül in Kombination mit einer weiteren Verbindung von therapeutischem Interesse oder mit einer Strahlentherapie verwendet wird.

## Claims

1. A peptide molecule capable of inhibiting the formation of the complex between Asf1 (anti-Silencing Factor 1) and histones H3-H4 and comprising elements E1-B-E2, wherein
a) E1 is a peptide of formula (I)
X₁-X₂-X₃-X₄-X₅-K-X₆-X₇-X₈- L-X₉-X₁₀-R-I-X₁₁ (I)
wherein
a1) either (SEQ ID No 1)
X₁ and X₂ are absent;
X₃ is I, R or L, preferably I or L;
X₄ is T or M, preferably T;
X₅ is P;
X₆ is E or D, preferably D;
X₇ is selected from the group consisting of R, A, I and E, preferably R, A and E; X₈ is selected from the group consisting of R, Q and E;
X₉ is R or A;
X₁₀ is R; and
X₁₁ is R or E;
a2) or (SEQ ID No 2)
X₁, X₇ and X₉ are A;
X₂ is S;
X₃ is T;
X₄, X₅, X₈ are E or R;
X₆ is W; and
X₁₀ is R;
and X₁₁ is R or A;
b) B is a peptide of formula (II)
G-X₁₂-G-X₁₃-X₁₄ (II) (SEQ ID No 3)
wherein
X₁₂ is A or P;
X₁₃ is absent or selected from the amino acids G, A and S;
X₁₄ is absent or S;
and
c) E2 is a peptide of formula (III)
X₁₅-X₁₆-X₁₇-X₁₈-G-X₁₉-X₂₀ (III) (SEQ ID No 4)
wherein
X₁₅ is R, V or A;
X₁₆ is T or V;
X₁₇ is L;
X₁₈ is Y, N or D;
X₁₉ is selected from the group consisting of F or M; and
X₂₀ is selected from the group consisting of G, Q and N.

2. A peptide molecule capable of inhibiting the formation of the complex between Asf1 (anti-Silencing Factor 1) and histones H3-H4 and comprising the elements E1-BE2, wherein the elements E1-B-E2 have one of the following sequences
X₃-X₄-P-K-X₆-X₇-X₈-L-X₉-R-R-I-X₁₁-G-X₁₂-G-X₁₃-X₁₄-X₁₅-X₁₆-L-X₁₈-G-X₁₉-X₂₀ (VIII) (SEQ ID No 11)
wherein
X₃ is I, R or L, preferably I or L;
X₄ is T or M, preferably T;
X₆ is E or D, preferably D;
X₇ is selected from the group consisting of R, A, I and E, preferably from R, A and E;
X₈ is selected from the group consisting of R, Q and E;
X₉ is R or A;
X₁₁ is R or E;
X₁₂ is A or P;
X₁₃ is absent or selected from the amino acids G, A and S;
X₁₄ is absent or S;
X₁₅ is R or V;
X₁₆ is T or V;
X₁₈ is Y, N or D;
X₁₉ is F, M, A or Q, preferably F or M, even more preferably F; and
X₂₀ is selected from the group consisting of G, Q and N;
A-S-T-X₄-R-K-W-A-E-L-A-R-R-I-R-G-X₁₂-G-X₁₃-X₁₄-X₁₅-X₁₆-L-X₁₈-G-X₁₉-X₂₀ (IX) (SEQ ID No 12)
wherein
X₄ is E or R;
X₁₂ is A or P;
X₁₃ is absent or selected from the amino acids G, A and S;
X₁₄ is absent or S;
X₁₅ is R or V;
X₁₆ is T or V;
X₁₈ is Y, N or D;
X₁₉ is F, M, A or Q, preferably F or M, even more preferably F; and
X₂₀ is selected from the group consisting of G, Q and N,
A-S-T-X₄-X₅-K-W-A-E-L-A-X₈-R-I-X₁₁-G-X₁₂-G-X₁₃-X₁₄-X₁₅-X₁₆-L-X₁₈-G-X₁₉-X₂₀ (XI) (SEQ ID No 82)
wherein
X₄, X₅, X₈ are E or R;
X₁₁ is R or A;
X₁₂ is A or P;
X₁₃ is absent or selected from the amino acids G, A and S;
X₁₄ is absent or S;
X₁₅ is R, V or A;
X₁₆ is T or V;
X₁₈ is Y, N or D;
X₁₉ is F or M, preferably F; and
X₂₀ is selected from the group consisting of G, Q and N.

3. The molecule according to any one of claims 1-2, wherein the elements E1-B-E2 have one of the following sequences
IMPKDIQLARRIRGAGGRTLYGFG (SEQ ID No 13)
IMPKDIQLARRIRGAGASRTLYGFG (SEQ ID No 14)
ITPKEAQLARRIRGAGGRTLNGFG (SEQ ID No 15)
ITPKEAQLARRIRGAGASRTLNGFG (SEQ ID No 16)
LTPKEAELARRIRGAGGRTLNGFG (SEQ ID No 17)
LTPKEAELARRIRGAGASRTLNGFG (SEQ ID No 18)
ITPKEAQLARRIEGAGASVTLNGFG (SEQ ID No 19)
LTPKEAELARRIEGAGASVTLNGFG (SEQ ID No 20)
ITPKEEQLRRRIEGAGASVTLNGFG (SEQ ID No 21)
ITPKEAQLARRIRGAGGVTLNGFG (SEQ ID No 22)
LTPKEAELARRIRGAGGVTLNGFG (SEQ ID No 23)
LTPKEAELARRIRGAGRTLNGFG (SEQ ID No 24)
LTPKEAELARRIRGAGGRTLNGFG (SEQ ID No 25)
LTPKEAELARRIRGAGASRTLNGFG (SEQ ID No 26)
RTPKERRLARRIRGAGGRTLNGFG (SEQ ID No 27)
RTPKEARLARRIRGAGGRTLNGFG (SEQ ID No 28)
LTPKEAELARRIRGAGGVTYDGFG (SEQ ID No 29)
LTPKEAELARRIRGAGGVTLNGAN (SEQ ID No 30)
STERKWAELARRIRGAGGVTLNGFG (SEQ ID No 31)
ASTERKWAELARRIRGAGGVTLNGFG (SEQ ID No 32)
ASTRRKWAELARRIRGAGGVTLNGFG (SEQ ID No 33)
ASTEEKWARLARRIRGAGGVTLNGFG (SEQ ID No 75)
ASTERKWAELARRIRGAGGVTLDGFG (SEQ ID No 76)
ASTERKWAELARRIRGAGGRTLNGFG (SEQ ID No 77)
ASTERKWAELARRIRGAGGATLNGFG (SEQ ID No 78)
ASTERKWAELARRIAGAGGVTLNGFG (SEQ ID No 79)
ASTEEKWARLARRIAGAGGVTLDGFG (SEQ ID No 80)
and a sequence having 90 or 95% identity therewith.

4. The molecule according to any one of claims 1-3, wherein the molecule further comprises a peptide facilitating cell penetration.

5. The molecule according to claim 4, wherein the peptide facilitating cell penetration is RRPRRPRRPRRPRRP (SEQ ID No 37).

6. The molecule according to any one of claims 1-5, the molecule comprising or consisting of a sequence selected from the group consisting of
GAMGTIMPKDIQLARRIRGAGGRTLYGFG (SEQ ID No 38)
GAMGTIMPKDIQLARRIRGAGASRTLYGFG (SEQ ID No 39)
GAMGTITPKEAQLARRIRGAGGRTLNGFG (SEQ ID No 40)
GAMGTITPKEAQLARRIRGAGASRTLNGFG (SEQ ID No 41)
GAMGTLTPKEAELARRIRGAGGRTLNGFG (SEQ ID No 42)
GAMGTLTPKEAELARRIRGAGASRTLNGFG (SEQ ID No 43)
GAMGTITPKEAQLARRIEGAGASVTLNGFG (SEQ ID No 44)
GAMGTLTPKEAELARRIEGAGASVTLNGFG (SEQ ID No 45)
GAMGTITPKEEQLRRRIEGAGASVTLNGFG (SEQ ID No 46)
GAMGTITPKEAQLARRIRGAGGVTLNGFG (SEQ ID No 47)
GAMGTLTPKEAELARRIRGAGGVTLNGFG (SEQ ID No 48)
GAMGTLTPKEAELARRIRGAGRTLNGFG (SEQ ID No 49)
GAMGTLTPKEAELARRIRGAGASRTLNGFG (SEQ ID No 51)
GAMGLTAAEHAKRSTLTPKEAQLARRIRGAGGVTLNGFG (SEQ ID No 52)
LTAAEHAKRSTLTPKEAELARRIRGAGGVTLNGFG (SEQ ID No 53)
LTAAEHAKRSTLTPKEAQLARRIEGAGASVTLNGFG (SEQ ID No 54)
LTAAEHAKRSTLTPKEAELARRIEGAGASVTLNGFG (SEQ ID No 55)
GAMGTRTPKERRLARRIRGAGGRTLNGFG (SEQ ID No 56)
GAMGTRTPKEARLARRIRGAGGRTLNGFG (SEQ ID No 57)
GRKKRRQRRRGAMGTITPKEAQLARRIRGAGGVTLNGFG (SEQ ID No 58)
RRPRRPRRPRRPRRPGAMGTITPKEAQLARRIRGAGGVTLNGFG (SEQ ID No 59)
GAMGTLTPKEAELARRIRGAGGVTYDGFG (SEQ ID No 61)
GAMGTLTPKEAELARRIRGAGGVTLNGFGASTG (SEQ ID No 62)
GAMGTLTPKEAELARRIRGAGGVTLNGANFVSTG (SEQ ID No 63)
GAMGRVPPAVRKLGNSTERKWAELARRIRGAGGVTLNGFG (SEQ ID No 64)
GAMGRVPPAVRKLGNASTERKWAELARRIRGAGGVTLNGFG (SEQ ID No 65)
ASTERKWAELARRIRGAGGVTLNGFG (SEQ ID No 32)
ASTRRKWAELARRIRGAGGVTLNGFG (SEQ ID No 33)
ASTEEKWARLARRIRGAGGVTLNGFG (SEQ ID No 75)
ASTERKWAELARRIRGAGGVTLDGFG (SEQ ID No 76)
ASTERKWAELARRIRGAGGRTLNGFG (SEQ ID No 77)
ASTERKWAELARRIRGAGGATLNGFG (SEQ ID No 78)
ASTERKWAELARRIAGAGGVTLNGFG (SEQ ID No 79)
ASTEEKWARLARRIAGAGGVTLDGFG (SEQ ID No 80)
RRPRRPRRPRRPRRPASTERKWAELARRIRGAGGVTLNGFGGCA (SEQ ID No 66)
RRPRRPRRPRRPRRPASTERKWAELARRIRGAGGVTLNGF G (SEQ ID No 67),
RRPRRPRRPRRPRRPGAMGTITPKEAQLARRIRGAGGVTLNGFGCA (SEQ ID No 50) and
RRPRRPRRPRRPRRPASTEEKWARLARRIAGAGGVTLDGFG (SEQ ID No 81)
and a sequence having 90 or 95% identity therewith.

7. The molecule according to any one of claims 1-6, the molecule comprising or consisting of a sequence selected from the group consisting of
RRPRRPRRPRRPRRPGAMGTITPKEAQLARRIRGAGGVTLNGFG (SEQ ID No 59)
RRPRRPRRPRRPRRPASTERKWAELARRIRGAGGVTLNGFGGCA (SEQ ID No 66)
RRPRRPRRPRRPRRPASTERKWAELARRIRGAGGVTLNGFG (SEQ ID No 67)
RRPRRPRRPRRPRRPGAMGTITPKEAQLARRIRGAGGVTLNGFGCA (SEQ ID No 50) and
RRPRRPRRPRRPRRPASTEEKWARLARRIAGAGGVTLDGFG (SEQ ID No 81).

8. The molecule according to any one of claims 1-7 as a medicament.

9. Pharmaceutical composition comprising a molecule according to any one of claims 1-8.

10. The pharmaceutical composition according to claim 9, wherein the composition comprises another compound of therapeutic interest.

11. The molecule according to any one of claims 1-7 for use in the treatment of cancer.

12. The molecule for use according to claim 11, the molecule being used in combination with another compound of therapeutic interest or with radiotherapy.
